# EUROPEAN PATENT APPLICATION

(11) **EP 0 623 621 A1**
(43) Date of publication of application: **09.11.1994**
(21) Application number: 94303063.5
(22) Date of filing: 21.04.1994
(51) Int. Cl.: C07D 498/08, A61K 31/535

(54) **Oxazabicyclo derivatives and their use as 5-HT4 receptor agonists**

(30) Priority: 30.04.1993 JP 103347/93
(71) Applicant: NISSHIN FLOUR MILLING CO., LTD., Chuo-ku, Tokyo 103 (JP)
(72) Inventor: Kikuchi, Haruhiko, c/o Nisshin Flour, Ohimachi, Iruma-gun, Saitama-ken (JP); Satoh, Hiroaki, c/o Nisshin Flour, Ohimachi, Iruma-gun, Saitama-ken (JP); Fukutomi, Ruta, c/o Nisshin Flour, Ohimachi, Iruma-gun, Saitama-ken (JP); Inomata, Kohei, c/o Nisshin Flour, Ohimachi, Iruma-gun, Saitama-ken (JP); Suzuki, Masashi, c/o Nisshin Flour, Ohimachi, Iruma-gun, Saitama-ken (JP); Ueno, Masahiro, c/o Nisshin Flour, Ohimachi, Iruma-gun, Saitama-ken (JP); Hagihara, Koichiro, c/o Nisshin Flour, Ohimachi, Iruma-gun, Saitama-ken (JP); Arai, Takeo, c/o Nisshin Flour, Ohimachi, Iruma-gun, Saitama-ken (JP); Eguchi, Haruko, c/o Nisshin Flour, Ohimachi, Iruma-gun, Saitama-ken (JP); Noguchi, Yumiko, c/o Nisshin Flour, Ohimachi, Iruma-gun, Saitama-ken (JP)
(74) Representative: Marshall, Monica Anne

(57) **Abstract**

Oxazabicyclo derivatives of formula (I): 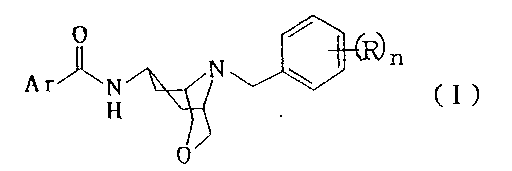
wherein the variable groups are as defined in the specification, which are useful as 5-HT₄ receptor agonist for the treatment of digestive tract diseases.

## Description

### FIELD OF THE INVENTION

This invention relates to new oxazabicyclo derivatives having 5-HT₄ receptor agonist activity, a process for their preparation and 5-HT₄ receptor agonists comprising those derivatives as an active ingredient.

### BACKGROUND OF THE INVENTION

The abnormality in function of a gastrointestinal motility by various causes such as chronic gastritis, gastrectomy, peptic ulcer results in the reflux of the gastric contents into the esophagus, delayed emptying of contents and the depression in the small and large intestinal function. This can lead to several gastrointestinal disorders including nausea, vomiting, heartburn, anorexia, abdominal distension, epigastric dysphoria, abdominalgia, and constipation and further reflux esophagitis. The diseases such as irritable bowel syndrome and spurious ileus is due to the depression in the gastrointestinal motility.

The agents for the treatment of these conditions and diseases include direct cholinergic agent (e.g., Aclatonium Napadisilate) or Dopamine antagonist (e.g., Domperidone). However, it is reported that these known agents have the problems in respect of pharmacological effects and side-effects including diarrhea and extrapyramidal syndrome.

Trends in Pharmacological Science, Vol. 13, 141-145, 1992 describes that the 5-HT₄ receptor exists in central and peripheral nervous systems and said receptor in the gastrointestinal nervous system participates in the release of acetylcholine from cholinergic nerve. Thus it is thought that the compounds acting on the 5-HT₄ receptor in alimentary canal and promoting the release of acetylcholine can be more effective gastroprokinetic agent with less side-effects. Further investigation of such compounds has been required.

### DETAILED DESCRIPTION OF THE INVENTION

We have now found that certain class of oxazabicyclo derivatives act as agonists at 5-HT₄ receptors and have a pharmaceutical use in the treatment of the digestive tract diseases.

Therefore, the present invention provides in one aspect a compound of formula (I) and pharmaceutically acceptable salts thereof. 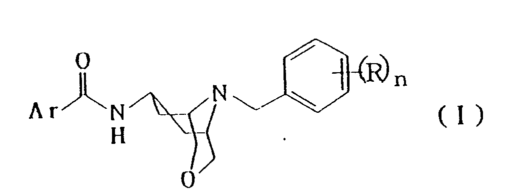
wherein R is a hydrogen atom, a halogen atom, a halo(C₁-C₆)alkyl group, a (C₁-C₆)alkoxy group, a nitro group, a hydroxyl group or an amino group, n is 1 or 2, the R groups being the same or different when n is 2, and Ar represents a radical of formula (II), (III), (IV), (V), (VI), (VII) or (VIII) 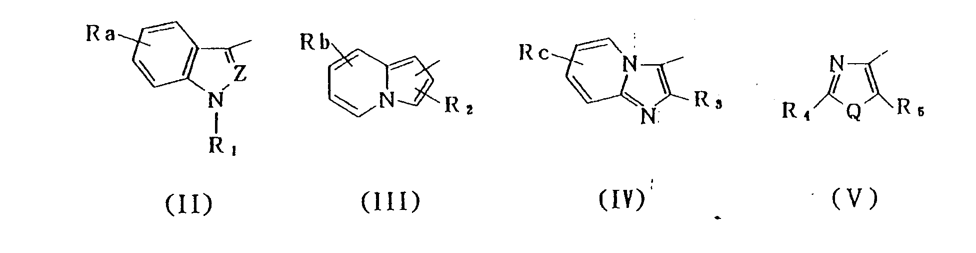 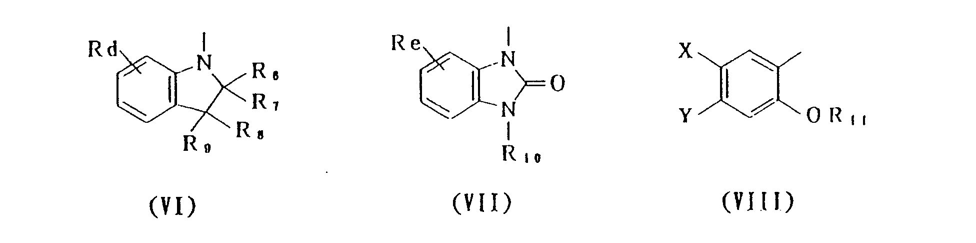
wherein
Ra to Re are independently a hydrogen atom, a halogen atom, a hydroxyl group, a (C₁-C₆)alkoxy group or a (C₁-C₈)alkyl group;
R₁ is a hydrogen atom, a (C₁-C₈)alkyl group, a (C₃-C₈)alkenyl group, a (C₃-C₈)alkynyl group, a (C₃-C₆)cycloalkyl group, a (C₃-C₆)cycloalkyl(C₁-C₆)alkyl group, a (C₁-C₆)alkoxy(C₂-C₆)alkyl group, a (C₃-C₆)oxoalkyl group, a (C₁-C₆)alkoxycarbonyl(C₁-C₆)alkyl group, a (C₁-C₆)alkoxycarbonyl group, a (C₁-C₆)alkanoyl group, a di(C₁-C₆)alkylamino(C₂-C₆)alkyl group, a hydroxy(C₂-C₆)alkyl group, a halo(C₁-C₆)alkyl group, a cyano(C₁-C₆)alkyl group, 4,6-diamino-2-triazinylmethyl group or a benzyl group optionally substituted by one or two substituents selected from the group consisting of halogen, C₁-C₆ alkoxy, nitro, hydroxyl and amino;
Z is CH or N;
R₂, R₃, R₅, R₆, R₉, R₁₀ and R₁₁ are independently a hydrogen atom or a (C₁-C₆)alkyl group;
R₄ is a (C₁-C₆)alkyl group, a pyridyl group or a phenyl group optionally substituted by halogen, C₁-C₆ alkyl, C₁-C₆ alkoxy or trifluoromethyl;
Q is N, S or O;
X is a halogen atom;
Y is NH₂ or a phthalimido group;
R₇ is a hydrogen atom;
R₈ is a hydrogen atom or a (C₁-C₄)alkyl group; or R₇ and R₈ together form a single bond.

The benzene ring in formula (I) may be unsubstituted or substituted by one or two substituents. Examples of the substituents include o-fluoro, m-fluoro, p-fluoro, o-chloro, m-chloro, p-chloro, 2,4-dichloro, 3,4-dichloro, trifluoromethyl, trichloromethyl, o-methoxy, m-methoxy, p-methoxy, o-ethoxy, p-ethoxy, o-propoxy, p-propoxy, o-butoxy, m-butoxy, p-butoxy, 3,4-dimethoxy, o-hydroxy, m-hydroxy, p-hydroxy, o-nitro, m-nitro, p-nitro, o-amino, m-amino and p-amino.

Referring to Ra, Rb, Rc, Rd and Re in formula (II), (III), (IV), (VI) and (VII), the halogen atom includes e.g. 5-fluoro, 6-fluoro, 7-fluoro, 5-chloro, 6-chloro and 7-chloro. The hydroxy group includes e.g. 5-hydroxy and 7-hydroxy. The (C₁-C₆)alkoxy group includes e.g. 5-methoxy, 7-methoxy, 5-ethoxy, 7-propoxy, 5-butoxy and 7-butoxy. The (C₁-C₈)alkyl group includes e.g. 5-methyl, 6-methyl, 7-methyl, 5-ethyl, 6-ethyl, 5-butyl and 7-butyl.

Referring to R₁ in formula (II), the (C₁-C₈)alkyl group includes e.g. methyl, ethyl, n-propyl, isopropyl, n-butyl, isobutyl, sec-butyl, n-pentyl, neopentyl and octyl. The (C₃-C₈)alkenyl group includes e.g. allyl, 1-propenyl, isopropenyl, 2-butenyl, 3-butenyl, 3-methyl-2-butenyl, 2-pentenyl, 3-pentenyl, 4-pentenyl, hexenyl and octenyl. The (C₃-C₈)alkynyl group includes e.g. 2-propynyl, butynyl and hexynyl. The (C₃-C₆)cycloalkyl group includes cyclopropyl, cyclobutyl, cyclopentyl and cyclohexyl. The (C₃-C₆)cycloalkyl(C₁-C₆)alkyl group includes e.g. cyclopropylmethyl, cyclopropylethyl, cyclopropylbutyl, cyclohexylmethyl, cyclohexylethyl and cyclohexylbutyl. The (C₁-C₆)alkoxy(C₂-C₆)alkyl group includes e.g. methoxyethyl and ethoxyethyl. The (C₃-C₆)oxoalkyl group includes e.g. 2-oxopropyl. The (C₁-C₆)alkoxycarbonyl(C₁-C₆)alkyl group includes e.g. methoxycarbonylmethyl, methoxycarbonylethyl, ethoxycarbonylmethyl and butoxycarbonylmethyl. The (C₁-C₆)alkoxycarbonyl group includes e.g. methoxycarbonyl and isobutoxy carbonyl. The (C₁-C₆)alkanoyl group includes e.g. acetyl, pivaloyl and hexanoyl. The di(C₁-C₆)alkylamino(C₂-C₆)alkyl group includes e.g. diethyl aminoethyl and dimethylaminoethyl. The hydroxy(C₂-C₆)alkyl group includes e.g. 2-hydroxyethyl and 2-hydroxypropyl. The halo(C₁-C₆)alkyl group includes e.g. 2-chloropropyl, 2-fluoroethyl, 2-bromoethyl and 2-iodoethyl. The substituted benzyl includes e.g. o-fluorobenzyl, m-fluorobenzyl, p-fluorobenzyl, o-chlorobenzyl, m-chlorobenzyl, p-chlorobenzyl, 2,4-dichlorobenzyl, 3,4-dichlorobenzyl, o-methoxybenzyl, m-methoxybenzyl, p-methoxybenzyl, o-ethoxybenzyl, o-butoxybenzyl, o-nitrobenzyl, m-nitrobenzyl, p-nitrobenzyl, 3,4-dimethoxybenzyl, o-hydroxybenzyl, m-hydroxybenzyl, p-hydroxybenzyl, o-aminobenzyl, m-aminobenzyl and p-aminobenzyl.

Referring to R₂, R₃, R₅, R₆, R₉, R₁₀ and R₁₁ in formulas (III), (IV), (V), (VI), (VII) and (VIII), the (C₁-C₆)alkyl group includes e.g. methyl, ethyl, n-propyl, isopropyl, n-butyl, isobutyl, sec-butyl, n-pentyl, neopentyl and n-hexyl.

When Ar represents formula (III), one of carbonyl in formula (I) and R₂ is attached to the i-position and the other is attached to the 3-position.

When Ar represents formula (V), the pyridyl group for R₄ includes e.g. 3-pyridyl and 4-pyridyl, the substituted phenyl group includes e.g. o-chlorophenyl, m-chlorophenyl, p-chlorophenyl, 3,5-dichlorophenyl, m-methylphenyl, p-methylphenyl, m-fluorophenyl, p-fluorophenyl, p-methoxyphenyl and m-trifluoromethylphenyl.

The compounds of formula (I) and their pharmaceutically acceptable salts including acid addition salts and quaternary ammonium salts may form hydrates or solvates which are included within the scope of the invention.

The compounds of formula (I) are prepared by a process which comprises oxidizing 2,5-dihydrofuran followed by reduction to give 3-oxa-1,5-pentanedial, reacting said pentanedial with unsubstituted or substituted benzylamine and acetonedicarboxylic acid to form a 9-substituted or unsubsittuted benzyl-3-oxa-9-azabicyclo[3.3.1]nonan-7-one, converting a carbonyl group on the oxazabicyclo ring to an oxime, reudcing the oxime to form a 7-amino-9-substituted or unsubstituted benzyl-3-oxa-9-azabicyclo[3.3.1]nonane and reacting the resulting amino compound with a compound represented by the formula ArCOOH wherein Ar is defined as above or its reactive derivatives. This series of reactions are shown by the following reaction scheme.

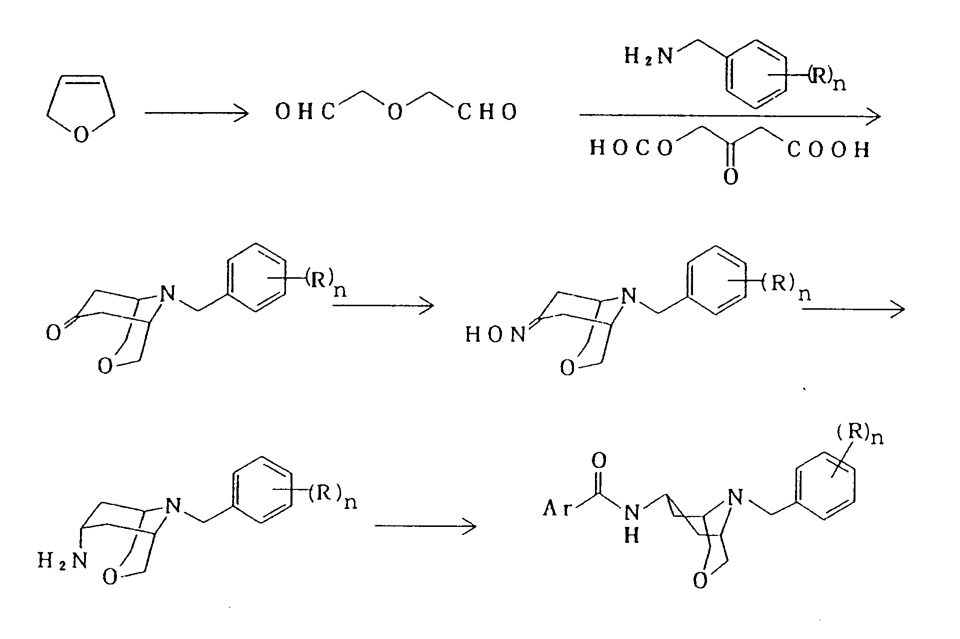

The reaction conditions used in the process of the present invention are described below.

3-Oxa-1,5-pentanedial can be prepared by subjecting 2,5-dihydrofuran to ozone oxidation followed by reduction with dimethyl sulfide. The ozone oxidation is performed by using an ozone gas in an amount of small excess to 10 moles per mole of 2,5-dihydrofuran, preferably 1.001-2 moles at a temperature between -100°C and room temperature, preferably -78°C to 0°C in the presence of a suitable solvent. The solvents include hydrocarbons such as pentane, hexane, heptane, cyclohexane, petroleum ether, benzene, halogen hydrocarbons such as carbon tetrachloride, chloroform, methylene chloride, ethers such as ethyl ether, THF, dioxane, esters such as ethyl acetate, acetone, DMF, nitromethane, acetic anhydride, carboxylic acids such as formic acid, acetic acid, alcohols such as methanol, ethanol, propanol, water. Methanol is preferred.

Following the ozone oxidation, dimethyl sulfide is added dropwise to a reaction vessel. Dimethyl sulfide is used in an amount of 1-10 moles per mole of 2,5-dihydrofuran, preferably 2-4 moles, added dropwise at a temperature of -100°C to a boiling point of the solvent, preferably -78°C and elevated to room temperature to carry out the reaction. The resulting 3-oxa-1,5-pentanedial is used for subsequent Robinson-Schöpf reaction with no further purification.

Robinson-Schöpf reaction is performed using a benzylamine derivative in an amount of 0.5 to 10 moles per mole of 2,5-dihydrofuran, preferably 1 to 3 moles and acetonedicarboxylic acid in an amount of 0.5 to 10 moles, preferably 1 to 3 moles, in the presence of a suitable solvent. By this reaction, 9-benzyl-3-oxa-9-azabicyclo[3.3.1]nonan-7-one derivatives are prepared. The reaction is performed at a temperature between a freezing point and a boiling point of the solvent, preferably 0 to 40°C. The solvents used include alcohols such as methanol, ethanol, propanol and water. In the reaction, carboxylic acids such as formic, acetic and citric acids, salts such as disodium hydrogenphosphate and sodium dihydrogenphosphate and bases such as sodium hydroxide, potassium hydroxide, triethylamine and pyridine may be added. The benzylamine derivatives may be added in the form of the hydrochloride.

9-Benzyl-3-oxa-9-azabicyclo[3.3.1]nonan-7-one oxime derivatives (oxime form) can be prepared by reacting 9-benzyl-3-oxa-9-azabicyclo[3.3.1]nonan-7-one derivatives with a hydroxylamine hydrochloride in the presence of a base. The bases includes e.g. pyridine, dimethylaminopyridine and triethylamine. The reaction is performed using the hydroxylamine hydrochloride in an amount of 0.5 to 10 moles per mole of 9-benzyl-3-oxa-9-azabicyclo[3.3.1]nonan-7-one derivatives, preferably 0.9 to 2 moles and the base in an amount of 0.5 to 10 moles, preferably 0.9 to 3 moles, at a temperature of 0°C to a boiling point of the solvent, preferably room temperature to a boiling point of the solvent. The solvents used include alcohols such as methanol, ethanol, propanol.

The synthesis of endo-7-amino-9-benzyl-3-oxa-9-azabicyclo[3.3.1]nonane derivatives can be prepared by reducing the oxime in a hydrogen gas atomosphere in the presence of Raney nickel. The reduction of the oxime is performed using ammonium acetate in an amount of 2 to 50 moles per mole of the oxime and Raney nickel in an amount of 0.01 to 10 times, preferably 0.02 to 2 times relative to the weight of the oxime. The hydrogen gas pressure ranges from normal pressure to 150 kg/cm², preferably 10 kg/cm² to 100 kg/cm². The reaction temperature ranges from room temperature to 200°C, preferably room temperature to 100°C. The solvents used include alcohols such as methanol, ethanol, propanol.

The compounds of formula (I) can be prepared by reacting endo-7-amino-9-benzyl-3-oxa-9-azabicyclo[3.3.1]nonane derivatives with the reactive derivatives of various carboxylic acids or reacting the carboxylic acids in the presence of a condensing agent such as carbodiimide derivatives.

The reaction of endo-7-amino-9-benzyl-3-oxa-9-azabicyclo[3.3.1]nonane derivatives with the reactive derivatives of carboxylic acid is performed using said derivatives in an amount of 0.1 to 10 moles, preferably 0.25 to 2 moles per mole of said nonane, at a temperature between a freezing point of the solvent and a boiling point of the solvent, preferably 0°C to 40°C. The solvents used include hydrocarbons such as pentane, hexane, heptane, cyclohexane, petroleum ether, benzene, halogen hydrocarbons such as carbon tetrachloride, chloroform, methylene chloride, ethers such as ethyl ether, THF, dioxane, esters such as ethyl acetate, acetone, DMF, nitromethane, DMSO, HMPA, pyridine. DMF and DMSO are preferable. In this reaction, bases such as dimethylaminopyridine, triethylamine, pyridine, potassium carbonate, sodium carbonate may be used.

Concrete examples of the carbodiimide derivatives include dicyclohexylcarbodiimide, 1-ethyl-3-(3'-dimethylaminopropyl)carbodiimide hydrochloride.

The condensation reaction of endo-7-amino-9-benzyl-3-oxa-9-azabicyclo[3.3.1]nonane derivatives with carboxylic acids is performed using carboxylic acids in an amount of 0.1 to 10 moles, preferably 0.5 to 2 moles per mole of endo-7-amino-9-benzyl-3-oxa-9-azabicyclo[3.3.1]nonane derivatives and using the carbodiimide derivatives in an amount of 0.1 to 10 moles, preferably 0.5 to 2 moles. The reaction can use optionally 0.5 to 2 moles of 1-hydroxybenzotriazole, N-hydroxysuccinimide. The reaction is performed at a temperature between a freezing point of the solvent and a boiling point of the solvent, preferably 0 to 40°C. The solvents used include hydrocarbons such as pentane, hexane, heptane, cyclohexane, petroleum ether, benzene, halogen hydrocarbons such as carbon tetrachloride, chloroform, methylene chloride, ethers such as ethyl ether, THF, dioxane, esters such as ethyl acetate, acetone, DMF, DMSO, nitromethane. Methylene chloride and DMF are preferable.

N-(endo-9-Benzyl-3-oxa-9-azabicyclo[3.3.1]non-7-yl)-1-R₁-indazole-3-carboxamide derivatives can be prepared by reacting N-(endo-9-benzyl-3-oxa-9-azabicyclo[3.3.1]non-7-yl)-1H-indazole-3-carboxamide derivatives with a R₁ halide in the presence of a base. The R₁ halides include e.g. methyl iodide, ethyl bromide, n-propyl bromide, isopropyl bromide, n-butyl bromide, isobutyl bromide, sec-butyl bromide, octyl bromide, allyl bromide, 3-methyl-2-butenyl bromide, bromomethylcyclopropane, bromocyclopropane, bromocyclobutane, bromocyclopentane, bromocyclohexane, p-fluorobenzylbromide. The bases include sodium hydride, n-butyl lithium.

The reaction of N-(endo-9-benzyl-3-oxa-9-azabicyclo[3.3.1]non-7-yl)-1H-indazole-3-carboxamide derivatives with the R₁ halide is performed using the base in an amount of 0.1 to 10 moles, preferably 0.8 to 1.2 moles per mole of N-(endo-9-benzyl-3-oxa-9-azabicyclo[3.3.1]non-7-yl)-1H-indazole-3-carboxamide derivatives and using the R₁ halide in an amount of 0.1 to 10 moles, preferably 0.5 to 3 moles.

The reaction is performed at a temperature between a freezing point of the solvent and a boiling point of the solvent, preferably 0 to 40°C. The solvents used include hydrcarbons such as pentane, hexane, heptane, cyclohexane, petroleum ether, benzene, ethers such as ethyl ether, THF, dioxane, esters such as ethyl acetate, DMF, DMSO. DMF is preferable.

N-(endo-9-Benzyl-3-oxa-9-azabicyclo[3.3.1]non-7-yl)-1-R₁-indazole-3-carboxamide derivatives can be prepared by condensing N-(endo-9-benzyl-3-oxa-9-azabicyclo[3.3.1]non-7-yl)-1H-indazole-3-carboxamide derivatives with R₁OH (J. Am. Chem. Soc. 104, 6876, 1982). R₁OH includes e.g. cyclopropanol, cyclobutanol, cyclopentanol, cyclohexanol.

The reaction is performed using diethyl azodicarboxylate in an amount of 0.5 to 2 moles, preferably 0.8 to 1.2 moles per mole of N-(endo-9-benzyl-3-oxa-9-azabicyclo[3.3.1]non-7-yl)-1H-indazole-3-carboxamide derivatives, triphenylphosphine (or tributylphosphine) in an amount of 0.5 to 2 moles, preferably 0.8 to 1.2 moles and R₁OH in an amount of 0.5 to 2 moles, preferably 0.8 to 1.2 moles. The reaction is performed at a temperature between a freezing point of the solvent and a boiling point of the solvent, preferably 0 to 100°C. Ethers such as ethyl ether, THF, dioxane, DMF are preferably used as the solvent.

Examples of the present compounds thus prepared are recited below.
N-[endo-9-(p-Fluorobenzyl)-3-oxa-9-azabicyclo[3.3.1]non-7-yl]-1H-indazole-3-carboxamide,
N-[endo-9-(p-Fluorobenzyl)-3-oxa-9-azabicyclo[3.3.1]non-7-yl]-1-methylindazole-3-carboxamide,
N-[endo-9-(p-Fluorobenzyl)-3-oxa-9-azabicyclo[3.3.1]non-7-yl]-1-ethylindazole-3-carboxamide,
N-[endo-9-(p-Fluorobenzyl)-3-oxa-9-azabicyclo[3.3.1]non-7-yl]-1-(n-propyl)indazole-3-carboxamide,
N-[endo-9-(p-Fluorobenzyl)-3-oxa-9-azabicyclo[3.3.1]non-7-yl]-1-(isopropyl)indazole-3-carboxamide,
N-[endo-9-(p-Fluorobenzyl)-3-oxa-9-azabicyclo[3.3.1]non-7-yl]-1-(n-butyl)indazole-3-carboxamide,
N-[endo-9-(p-Fluorobenzyl)-3-oxa-9-azabicyclo[3.3.1]non-7-yl]-1-(isobutyl)indazole-3-carboxamide,
N-[endo-9-(p-Fluorobenzyl)-3-oxa-9-azabicyclo[3.3.1]non-7-yl]-1-(sec-butyl)indazole-3-carboxamide,
N-[endo-9-(p-Fluorobenzyl)-3-oxa-9-azabicyclo[3.3.1]non-7-yl]-1-octylindazole-3-carboxamide,
N-[endo-9-(p-Fluorobenzyl)-3-oxa-9-azabicyclo[3.3.1]non-7-yl]-1-allylindazole-3-carboxamide,
N-[endo-9-(p-Fluorobenzyl)-3-oxa-9-azabicyclo[3.3.1]non-7-yl]-1-(1-propenyl)indazole-3-carboxamide,
N-[endo-9-(p-Fluorobenzyl)-3-oxa-9-azabicyclo[3.3.1]non-7-yl]-1-(3-methyl-2-butenyl)indazole-3-carboxamide,
N-[endo-9-(p-Fluorobenzyl)-3-oxa-9-azabicyclo[3.3.1]non-7-yl]-1-(cyclopropylmethyl)indazole-3-carboxamide,
N-[endo-9-(p-Fluorobenzyl)-3-oxa-9-azabicyclo[3.3.1]non-7-yl]-1-cyclopropylindazole-3-carboxamide,
N-[endo-9-(p-Fluorobenzyl)-3-oxa-9-azabicyclo[3.3.1]non-7-yl]-1-cyclobutylindazole-3-carboxamide,
N-[endo-9-(p-Fluorobenzyl)-3-oxa-9-azabicyclo[3.3.1]non-7-yl]-1-cyclobutylindazole-3-carboxamide,
N-[endo-9-(p-Fluorobenzyl)-3-oxa-9-azabicyclo[3.3.1]non-7-yl]-1-cyclopentylindazole-3-carboxamide,
N-[endo-9-(p-Fluorobenzyl)-3-oxa-9-azabicyclo[3.3.1]non-7-yl]-1-cyclohexylindazole-3-carboxamide,
N-[endo-9-(p-Fluorobenzyl)-3-oxa-9-azabicyclo[3.3.1]non-7-yl]-1-(2-oxopropyl)indazole-3-carboxamide,
N-[endo-9-(p-Fluorobenzyl)-3-oxa-9-azabicyclo[3.3.1]non-7-yl]-1-(2-hydroxypropyl)indazole-3-carboxamide,
N-[endo-9-(p-Fluorobenzyl)-3-oxa-9-azabicyclo[3.3.1]non-7-yl]-1-(2-chloropropyl)indazole-3-carboxamide,
N-[endo-9-(p-Fluorobenzyl)-3-oxa-9-azabicyclo[3.3.1]non-7-yl]-1-(p-fluorobenzyl)indazole-3-carboxamide,
N-[endo-9-(p-Fluorobenzyl)-3-oxa-9-azabicyclo[3.3.1]non-7-yl]-4-amino-5-chloro-2-methoxybenzamide,
N-[endo-9-(p-Fluorobenzyl)-3-oxa-9-azabicyclo[3.3.1]non-7-yl]-4-amino-5-chloro-2-ethoxybenzamide,
N-[endo-9-(p-Fluorobenzyl)-3-oxa-9-azabicyclo[3.3.1]non-7-yl]-4-phthalimido-5-chloro-2-methoxybenzamide,
N-[endo-9-Benzyl-3-oxa-9-azabicyclo[3.3.1]non-7-yl]-1H-indazole-3-carboxamide,
N-[endo-9-Benzyl-3-oxa-9-azabicyclo[3.3.1]non-7-yl]-1-allylindazole-3-carboxamide,
N-[endo-9-Benzyl-3-oxa-9-azabicyclo[3.3.1]non-7-yl]-1-(3-methyl-2-butenyl)indazole-3-carboxamide,
N-[endo-9-Benzyl-3-oxa-9-azabicyclo[3.3.1]non-7-yl]-1-(cyclopropylmethyl)indazole-3-carboxamide,
N-[endo-9-(o-Fluorobenzyl)-3-oxa-9-azabicyclo[3.3.1]non-7-yl]-1H-indazole-3-carboxamide,
N-[endo-9-(o-Fluorobenzyl)-3-oxa-9-azabicyclo[3.3.1]non-7-yl]-1-(n-propyl)indazole-3-carboxamide,
N-[endo-9-(o-Fluorobenzyl)-3-oxa-9-azabicyclo[3.3.1]non-7-yl]-1-(n-butyl)indazole-3-carboxamide,
N-[endo-9-(o-Fluorobenzyl)-3-oxa-9-azabicyclo[3.3.1]non-7-yl]-1-(3-methyl-2-butenyl)indazole-3-carboxamide,
N-[endo-9-(o-Fluorobenzyl)-3-oxa-9-azabicyclo[3.3.1]non-7-yl]-1-(cyclopropylmethyl)indazole-3-carboxamide,
N-[endo-9-(m-Fluorobenzyl)-3-oxa-9-azabicyclo[3.3.1]non-7-yl]-1H-indazole-3-carboxamide,
N-[endo-9-(m-Fluorobenzyl)-3-oxa-9-azabicyclo[3.3.1]non-7-yl]-1-allylindazole-3-carboxamide,
N-[endo-9-(m-Fluorobenzyl)-3-oxa-9-azabicyclo[3.3.1]non-7-yl]-1-(3-methyl-2-butenyl)indazole-3-carboxamide,
N-[endo-9-(m-Fluorobenzyl)-3-oxa-9-azabicyclo[3.3.1]non-7-yl]-1-(cyclopropylmethyl)indazole-3-carboxamide,
N-[endo-9-(o-Chlorobenzyl)-3-oxa-9-azabicyclo[3.3.1]non-7-yl]-1-(3-methyl-2-butenyl)indazole-3-carboxamide,
N-[endo-9-(m-Chlorobenzyl)-3-oxa-9-azabicyclo[3.3.1]non-7-yl]-1-(3-methyl-2-butenyl)indazole-3-carboxamide,
N-[endo-9-(p-Chlorobenzyl)-3-oxa-9-azabicyclo[3.3.1]non-7-yl]-1H-indazole-3-carboxamide,
N-[endo-9-(p-Chlorobenzyl)-3-oxa-9-azabicyclo[3.3.1]non-7-yl]-1-allylindazole-3-carboxamide,
N-[endo-9-(p-Chlorobenzyl)-3-oxa-9-azabicyclo[3.3.1]non-7-yl]-1-(3-methyl-2-butenyl)indazole-3-carboxamide,
N-[endo-9-(p-Chlorobenzyl)-3-oxa-9-azabicyclo[3.3.1]non-7-yl]-1-(cyclopropylmethyl)indazole-3-carboxamide,
N-[endo-9-(2,4-Dichlorobenzyl)-3-oxa-9-azabicyclo[3.3.1]non-7-yl]-1H-indazole-3-carboxamide,
N-[endo-9-(2,4-Dichlorobenzyl)-3-oxa-9-azabicyclo[3.3.1]non-7-yl]-1-allylindazole-3-carboxamide,
N-[endo-9-(2,4-Dichlorobenzyl)-3-oxa-9-azabicyclo[3.3.1]non-7-yl]-1-(cyclopropylmethyl)indazole-3-carboxamide,
N-[endo-9-(2,4-Dichlorobenzyl)-3-oxa-9-azabicyclo[3.3.1]non-7-yl]-1-(3-methyl-2-butenyl)indazole-3-carboxamide,
N-[endo-9-(3,4-Dichlorobenzyl)-3-oxa-9-azabicyclo[3.3.1]non-7-yl]-1H-indazole-3-carboxamide,
N-[endo-9-(3,4-Dichlorobenzyl)-3-oxa-9-azabicyclo[3.3.1]non-7-yl]-1-allylindazole-3-carboxamide,
N-[endo-9-(3,4-Dichlorobenzyl)-3-oxa-9-azabicyclo[3.3.1]non-7-yl]-1-(1-propenyl)indazole-3-carboxamide,
N-[endo-9-(3,4-Dichlorobenzyl)-3-oxa-9-azabicyclo[3.3.1]non-7-yl]-1-(3-methyl-2-butenyl)indazole-3-carboxamide,
N-[endo-9-(3,4-Dichlorobenzyl)-3-oxa-9-azabicyclo[3.3.1]non-7-yl]-1-(cyclopropylmethyl)indazole-3-carboxamide,
N-[endo-9-(p-Trifluoromethylbenzyl)-3-oxa-9-azabicyclo[3.3.1]non-7-yl]-1H-indazole-3-carboxamide,
N-[endo-9-(p-Trifluoromethylbenzyl)-3-oxa-9-azabicyclo[3.3.1]non-7-yl]-1-allylindazole-3-carboxamide,
N-[endo-9-(p-Trifluoromethylbenzyl)-3-oxa-9-azabicyclo[3.3.1]non-7-yl]-1-(3-methyl-2-butenyl)indazole-3-carboxamide,
N-[endo-9-(p-Trifluoromethylbenzyl)-3-oxa-9-azabicyclo[3.3.1]non-7-yl]-1-(cyclopropylmethyl)indazole-3-carboxamide,
N-[endo-9-(o-Methoxybenzyl)-3-oxa-9-azabicyclo[3.3.1]non-7-yl]-1H-indazole-3-carboxamide,
N-[endo-9-(o-Methoxybenzyl)-3-oxa-9-azabicyclo[3.3.1]non-7-yl]-1-(n-propyl)indazole-3-carboxamide,
N-[endo-9-(o-Methoxybenzyl)-3-oxa-9-azabicyclo[3.3.1]non-7-yl]-1-allylindazole-3-carboxamide,
N-[endo-9-(o-Methoxybenzyl)-3-oxa-9-azabicyclo[3.3.1]non-7-yl]-1-(3-methyl-2-butenyl)indazole-3-carboxamide,
N-[endo-9-(o-Methoxybenzyl)-3-oxa-9-azabicyclo[3.3.1]non-7-yl]-1-cyclopentylindazole-3-carboxamide,
N-[endo-9-(m-Methoxybenzyl)-3-oxa-9-azabicyclo[3.3.1]non-7-yl]-1H-indazole-3-carboxamide,
N-[endo-9-(m-Methoxybenzyl)-3-oxa-9-azabicyclo[3.3.1]non-7-yl]-1-(n-propyl)indazole-3-carboxamide,
N-[endo-9-(m-Methoxybenzyl)-3-oxa-9-azabicyclo[3.3.1]non-7-yl]-1-allylindazole-3-carboxamide,
N-[endo-9-(m-Methoxybenzyl)-3-oxa-9-azabicyclo[3.3.1]non-7-yl]-1-(3-methyl-2-butenyl)indazole-3-carboxamide,
N-[endo-9-(m-Methoxybenzyl)-3-oxa-9-azabicyclo[3.3.1]non-7-yl]-1-cyclopentylindazole-3-carboxamide,
N-[endo-9-(p-Methoxybenzyl)-3-oxa-9-azabicyclo[3.3.1]non-7-yl]-1H-indazole-3-carboxamide,
N-[endo-9-(p-Methoxybenzyl)-3-oxa-9-azabicyclo[3.3.1]non-7-yl]-1-methylindazole-3-carboxamide,
N-[endo-9-(p-Methoxybenzyl)-3-oxa-9-azabicyclo[3.3.1]non-7-yl]-1-ethylindazole-3-carboxamide,
N-[endo-9-(p-Methoxybenzyl)-3-oxa-9-azabicyclo[3.3.1]non-7-yl]-1-(n-propyl)indazole-3-carboxamide,
N-[endo-9-(p-Methoxybenzyl)-3-oxa-9-azabicyclo[3.3.1]non-7-yl]-1-(isopropyl)indazole-3-carboxamide,
N-[endo-9-(p-Methoxybenzyl)-3-oxa-9-azabicyclo[3.3.1]non-7-yl]-1-(n-buty)indazole-3-carboxamide,
N-[endo-9-(p-Methoxybenzyl)-3-oxa-9-azabicyclo[3.3.1]non-7-yl]-1-(isobutyl)indazole-3-carboxamide,
N-[endo-9-(p-Methoxybenzyl)-3-oxa-9-azabicyclo[3.3.1]non-7-yl]-1-(sec-butyl)indazole-3-carboxamide,
N-[endo-9-(p-Methoxybenzyl)-3-oxa-9-azabicyclo[3.3.1]non-7-yl]-1-octylindaozle-3-carboxamide,
N-[endo-9-(p-Methoxybenzyl)-3-oxa-9-azabicyclo[3.3.1]non-7-yl]-1-allylindazole-3-carboxamide,
N-[endo-9-(p-Methoxybenzyl)-3-oxa-9-azabicyclo[3.3.1]non-7-yl]-1-(n-propenyl)indazole-3-carboxamide,
N-[endo-9-(p-Methoxybenzyl)-3-oxa-9-azabicyclo[3.3.1]non-7-yl]-1-(3-methyl-2-butenyl)indazole-3-carboxamide,
N-[endo-9-(p-Methoxybenzyl)-3-oxa-9-azabicyclo[3.3.1]non-7-yl]-1-(cyclopropylmethyl)indazole-3-carboxamide,
N-[endo-9-(p-Methoxybenzyl)-3-oxa-9-azabicyclo[3.3.1]non-7-yl]-1-cyclopropylindazole-3-carboxamide,
N-[endo-9-(p-Methoxybenzyl)-3-oxa-9-azabicyclo[3.3.1]non-7-yl]-1-cyclobutylindazole-3-carboxamide,
N-[endo-9-(p-Methoxybenzyl)-3-oxa-9-azabicyclo[3.3.1]non-7-yl]-1-cyclopentylindazole-3-carboxamide,
N-[endo-9-(p-Methoxybenzyl)-3-oxa-9-azabicyclo[3.3.1]non-7-yl]-1-cyclohexylindazole-3-carboxamide,
N-[endo-9-(p-Methoxybenzyl)-3-oxa-9-azabicyclo[3.3.1]non-7-yl]-1-(2-ethoxyethyl)indazole-3-carboxamide,
N-[endo-9-(p-Methoxybenzyl)-3-oxa-9-azabicyclo[3.3.1]non-7-yl]-1-(2-oxopropyl)indazole-3-carboxamide,
N-[endo-9-(p-Methoxybenzyl)-3-oxa-9-azabicyclo[3.3.1]non-7-yl]-1-(2-hydroxypropyl)indazole-3-carboxamide,
N-[endo-9-(p-Methoxybenzyl)-3-oxa-9-azabicyclo[3.3.1]non-7-yl]-1-(2-chloropropyl)indazole-3-carboxamide,
N-[endo-9-(p-Methoxybenzyl)-3-oxa-9-azabicyclo[3.3.1]non-7-yl]-1-(p-fluorobenzyl)indazole-3-carboxamide,
N-[endo-9-(p-Methoxybenzyl)-3-oxa-9-azabicyclo[3.3.1]non-7-yl]-4-amino-5-chloro-2-methoxybenzamide,
N-[endo-9-(p-Methoxybenzyl)-3-oxa-9-azabicyclo[3.3.1]non-7-yl]-4-amino-5-chloro-2-ethoxybenzamide,
N-[endo-9-(p-Methoxybenzyl)-3-oxa-9-azabicyclo[3.3.1]non-7-yl]-4-phthalimido-5-chloro-2-methoxybenzamide,
N-[endo-9-(o-Nitrobenzyl)-3-oxa-9-azabicyclo[3.3.1]non-7-yl]-1-(3-methyl-2-butenyl)indazole-3-carboxamide,
N-[endo-9-(m-Nitrobenzyl)-3-oxa-9-azabicyclo[3.3.1]non-7-yl]-1-(3-methyl-2-butenyl)indazole-3-carboxamide,
N-[endo-9-(p-Nitrobenzyl)-3-oxa-9-azabicyclo[3.3.1]non-7-yl]-1-(3-methyl-2-butenyl)indazole-3-carboxamide,
N-[endo-9-(3,4-Dimethoxybenzyl)-3-oxa-9-azabicyclo[3.3.1]non-7-yl]-1-(3-methyl-2-butenyl)indazole-3-carboxamide,
N-[endo-9-(o-Hydroxybenzyl)-3-oxa-9-azabicyclo[3.3.1]non-7-yl]-1-(3-methyl-2-butenyl)indazole-3-carboxamide,
N-[endo-9-(m-Hydroxybenzyl)-3-oxa-9-azabicyclo[3.3.1]non-7-yl]-1-(3-methyl-2-butenyl)indazole-3-carboxamide,
N-[endo-9-(p-Hydroxybenzyl)-3-oxa-9-azabicyclo[3.3.1]non-7-yl]-1-(3-methyl-2-butenyl)indazole-3-carboxamide,
N-[endo-9-(o-Aminobenzyl)-3-oxa-9-azabicyclo[3.3.1]non-7-yl]-1-(3-methyl-2-butenyl)indazole-3-carboxamide,
N-[endo-9-(m-Aminobenzyl)-3-oxa-9-azabicyclo[3.3.1]non-7-yl]-1-(3-methyl-2-butenyl)indazole-3-carboxamide,
N-[endo-9-(p-Aminobenzyl)-3-oxa-9-azabicyclo[3.3.1]non-7-yl]-1-(3-methyl-2-butenyl)indazole-3-carboxamide,
N-[endo-9-(p-Fluorobenzyl)-3-oxa-9-azabicyclo[3.3.1]non-7-yl]-1-(3-methyl-2-butenyl)-5-methoxyindazole-3-carboxamide,
N-[endo-9-(p-Fluorobenzyl)-3-oxa-9-azabicyclo[3.3.1]non-7-yl]-1-(3-methyl-2-butenyl)-5-methoxyindazole-3-carboxamide,
N-[endo-9-(p-Fluorobenzyl)-3-oxa-9-azabicyclo[3.3.1]non-7-yl]-1-(3-methyl-2-butenyl)-5-chloroindazole-3-carboxamide,
N-[endo-9-(p-Fluorobenzyl)-3-oxa-9-azabicyclo[3.3.1]non-7-yl]-1-(3-methyl-2-butenyl)-5-hydroxyindazole-3-carboxamide,
N-[endo-9-(p-Fluorobenzyl)-3-oxa-9-azabicyclo[3.3.1]non-7-yl]-1-(3-methyl-2-butenyl)indazole-3-carboxamide,
N-[endo-9-(p-Fluorobenzyl)-3-oxa-9-azabicyclo[3.3.1]non-7-yl]-3-methylindolizine-1-carboxamide,
N-[endo-9-(p-Fluorobenzyl)-3-oxa-9-azabicyclo[3.3.1]non-7-yl]-1-ethylindolizine-1-carboxamide,
N-[endo-9-(p-Fluorobenzyl)-3-oxa-9-azabicyclo[3.3.1]non-7-yl]-2-methylimidazo[1,2-a]pyridine-3-carboxamide,
N-[endo-9-(p-Fluorobenzyl)-3-oxa-9-azabicyclo[3.3.1]non-7-yl]-2-phenyl-4-thiazolecarboxamide,
N-[endo-9-(p-Fluorobenzyl)-3-oxa-9-azabicyclo[3.3.1]non-7-yl]-2-phenyl-4-oxazolecarboxamide,
N-[endo-9-(p-Fluorobenzyl)-3-oxa-9-azabicyclo[3.3.1]non-7-yl]-3, 3-dimethyl-1-indolinecarboxamide,
N-[endo-9-(p-Fluorobenzyl)-3-oxa-9-azabicyclo[3.3.1]non-7-yl]-1-indolecarboxamide, and
N-[endo-9-(p-Fluorobenzyl)-3-oxa-9-azabicyclo[3.3.1]non-7-yl]-3-methyl-2-oxo-2,3-dihydrobenzimidazole-1-carboxamide.

The pharmaceutically acceptable salts of the compounds of formula (I) include acid addition salts with pharmaceutically acceptable inorganic acids such as hydrochloric, hydrobromic, phosphoric, sulfuric acids, and pharmaceutically acceptable organic acids such as acetic, tartaric, maleic, citric, succinic and oxalic acids.

The compounds of the present invention acting on 5-HT₄ receptors possess a potent gastrointestinal motility-accelerating activity as demonstrated by the examples which are given later, and are useful in the treatment of the digestive tract diseases. Examples of those diseases include chronic gastritis, postgastrectomy syndrome, gastrointestinal symptoms associated with peptic ulcer, reflux esophagitis, irritable bowel syndrome and spurious ileus.

Gastrointestinal motility is regulated by sympathetic and parasympathetic nervous systmes. In parasymphathetic nervous system, acetylcholine is one of the most important neurotransmitters in regulating the motility. A release of acethylcholine from the enteric neurons in myenteric plexus makes a contraction of gastrointestinal tract. Therefore, stimulating the release of acetylcholine from enteric neurons accelerates the gastrointestinal motility.

The existence of 5-HT₄ receptors was demonstrated in gastrointestinal tract (Craig and Clark, J. Pharmacol. Exper. Ther. 252, 1378-1386, 1990; Elswood et al., Eur. J. Pharmacol. 196, 149-155, 1991; Baxter et al., Naunyn-Schmiedeberg's Arch. Pharmacol. 343; 439-446, 1991). Since it is reported that 5-HT₄ receptor regulates the release of acetylcholine in enteric neurons (Kilbinger and Wolf, Naunyn-Schmiedeberg's Arch Pharmacol. 345: 270-275, 1992), an agent acting on the 5-HT₄ receptor and stimulating the release of acetylcholine is found useful as a gastroprokinetic agent in treatment of gastrointestinal motility disorders.

The invention provides in another aspect a 5-HT₄ receptor agonist, which comprises a compound of formula (I) or a pharmaceutically acceptable salt thereof.

The invention also provides a pharmaceutical composition comprising as an active ingredient a compound of formula (I) or a pharmaceutically acceptable salt thereof, and a pharmaceutically acceptable carrier.

The compounds of the invention can usually be administered orally or parenterally in the form of a pharmaceutical preparation. The pharmaceutical preparations include e.g. tablets, powders, granules, sugar-coated tablets, hard and soft capsules, solutions, emulsions and suspensions for oral administeration and injections for parenteral administration. These preparations can be prepared by conventional methods employing conventional additives such as excipients, stabilizers, antioxidants, solubilizers, wetting agents, emulsifiers, lubricants, colorants, flavorings, buffers, preservatives or the like.

The invention further provides a method for the treatment or prophylaxis of the digestive tract diseases such as chronic gastritis, postgastrectomy syndrome, gastrointestinal symptoms associated with peptic ulcer, reflux esophagitis, irritable bowel syndrome and spurious ileus, which comprises administering to a subject in need of such treatment a compound of formula (I) or a pharmaceutically acceptable salt thereof.

Route and dosage of administration for the compounds of the invention are not specifically limited and are appropriately chosen depending upon form of the formulation, age and sex of the patient, severity of the disease and other factors. Daily dosage of the active ingredient is 0.1 to 100 mg. No adverse toxicological effects are indicated at any of the above dosage ranges.

The invention is further illustrated by the following examples.

### Preparative Example 1

Synthesis of 3-oxa-1,5-pentanedial

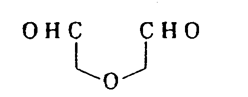

An ozone gas was introduced at -78°C to a solution of 2,5-dihydrofuran (20 g) in methanol (200 ml) for 6 hrs. A nitrogen gas was introduced at the same temperature to the solution, dimethyl sulfide (46.1 ml) was added dropwise over a period of 15 minutes and the mixture was stirred for 30 minutes. The reaction solution was elevated to -30°C and stirred for 30 minutes. Further stirring was continued at 0°C for 30 minutes and at room temperature for 30 minutes. The solvent was distilled off under reduced pressure to afford crude 3-oxa-1,5-pentanedial. This product was used for the subsequent reaction with no further purification.

### Preparative Example 2

Synthesis of 9-p-fluorobenzyl-3-oxa-9-azabicyclo[3.3.1]nonan-7-one

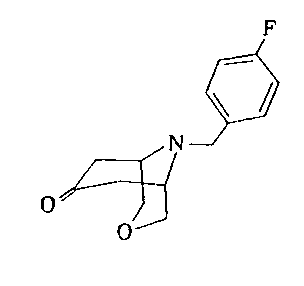

To a solution of disodium hydrogen phosphate, dodecahydrate (104.2 g) and citric acid (26.32 g) in water (400 ml) were added in turn p-fluorobenzylamine hydrochloride (74 g) and acetone dicarboxylic acid (66.9 g) and adjusted to pH 4.6 with 10% aqueous sodium hydroxide solution. To the reaction solution was added dropwise at room temperature a solution of crude 3-oxa-1,5-pentanedial (30 g) in methanol (20 ml) and stirred for 65 hrs. Then, 10% aqueous sodium hydroxide solution (300 ml) was added to the reaction solution, which was extracted three times with chloroform. The combined organic layer was washed with 10% aqueous sodium hydroxide solution (200 ml), dried over potassium carbonate and the solvent was distilled off under reduced pressure. Purification of the residue by silica gel column chromatography (2/1 hexane/ethyl acetate) gave 9-p-fluorobenzyl-3-oxa-9-azabicyclo[3.3.1]nonan-7-one (31.4 g).
¹H NMR(CDCl₃) δ 2.33(d, J=16Hz, 2H), 2.72(dd, J=6Hz, 16Hz, 2H), 3.13(d, J=6Hz, 2H), 3.72(d, J=11Hz, 2H), 3.82(d, J=11Hz, 2H), 7.03(d, J=9Hz, 1H), 7.05(d, J=8Hz, 1H), 7.36(d, J=8Hz, 1H), 7.38(d, J=8Hz, 1H)

The following compounds were prepared in a similar manner.
9-(m-Fluorobenzyl)-3-oxa-9-azabicyclo[3.3.1]nonan-7-one
¹H NMR(CDCl₃) δ 2.35(d, J=16Hz, 2H), 2.71(dd, J=6Hz, 16Hz, 2H), 3.15(d, J=6Hz, 2H), 3.73(d, J=11Hz, 2H), 3.84(d, J=11Hz, 2H), 3.91(s, 2H), 6.98(td, J=8Hz, 2Hz, 1H), 7.14-7.17(m, 2H), 7.28-7.52(m, 1H)
9-(o-Fluorobenzyl)-3-oxa-9-azabicyclo[3.3.1]nonan-7-one
¹H NMR(CDCl₃) δ 2.35(d, J=16Hz, 2H), 2.78(dd, J=6Hz, 16Hz, 2H), 3.17(d, J=6Hz, 2H), 3.72(d, J=11Hz, 2H), 3.83(d, J=11Hz, 2H), 3.94(s, 2H), 7.06(t, J=9Hz, 1H), 7.15(t, J=7Hz, 1H), 7.25-7.31(m, 1H), 7.50(td, J=7Hz, 1Hz, 1H)
9-(2,4-Dichlorobenzyl)-3-oxa-9-azabicyclo[3.3.1]nonan-7-one
¹H NMR(CDCl₃) δ 2.37(d, J=16Hz, 2H), 2.76(dd, J=6Hz, 16Hz, 2H), 3.14(d, J=6Hz, 2H), 3.73(d, J=11Hz, 2H), 3.84(d, J=11Hz, 2H), 3.96(s, 2H), 7.26(dd, J=2Hz, 8Hz, 1H), 7.40(d, 2Hz, 1H), 7.50(d, J=8Hz, 1H)
9-(3,4-Dichlorobenzyl)-3-oxa-9-azabicyclo[3.3.1]nonan-7-one
¹H NMR(CDCl₃) δ 2.35(d, J=16Hz, 2H), 2.70(dd, J=5Hz, 16Hz, 2H), 3.13(d, J=5Hz, 2H), 3.73(d, J=11Hz, 2H), 3.84(d, J=11Hz, 2H), 3.86(s, 2H), 7.24(dd, J=2Hz, 8Hz, 1H), 7.42(d, J=8Hz, 1H), 7.52(d, J=2Hz, 1H)
9-(p-Trifluoromethylbenzyl)-3-oxa-9-azabicyclo[3.3.1]nonan-7-one
¹H NMR(CDCl₃) δ 2.35(d, J=16Hz, 2H), 2.73(dd, J=5Hz, 16Hz, 2H), 3.14(d, J=6Hz, 2H), 3.73(d, J=11Hz, 2H), 3.85(d, J=11Hz, 2H), 3.97(s, 2H), 7.54(d, J=8Hz, 2H), 7.62(d, J=8Hz, 2H)
9-Benzyl-3-oxa-9-azabicyclo[3.3.1]nonan-7-one
¹H NMR(CDCl₃) δ 2.40(d, J=16Hz, 2H), 2.74(dd, J=6Hz, 16Hz, 2H), 3.16(d, J=6Hz, 2H), 3.71(d, J=11Hz, 2H), 3.83(d, J=11Hz, 2H), 3.91(s, 2H), 7.26-7.42(m, 5H)
9-(p-Methoxybenzyl)-3-oxa-9-azabicyclo[3.3.1]nonan-7-one
¹H NMR(CDCl₃) δ 2.31(d, J=16Hz, 2H), 2.72(dd, J=6Hz, 16Hz, 2H), 3.15(d, J=5Hz, 2H), 3.70(d, J=11Hz, 2H), 3.79(d, J=11Hz, 2H), 3.81(s, 3H), 3.84(s, 2H), 6.88(d, J=9Hz, 2H), 7.31(d, J=9Hz, 2H)

### Preparative Example 3

Synthesis of 9-p-fluorobenzyl-3-oxa-9-azabicyclo[3.3.1]nonan-7-one oxime

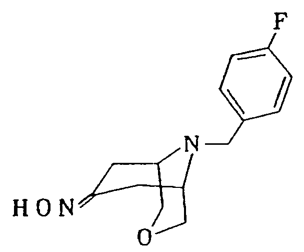

To a solution of 9-p-fluorobenzyl-3-oxa-9-azabicyclo[3.3.1]nonan-7-one (6.35 g) in ethanol (60 ml) were added in turn pyridine (3.0 ml) and hydroxylamine hydrochloride (1.95 g) and heated under reflux for 30 minutes. To the reaction solution was added 10% aqueous sodium hydroxide solution (20 ml) and ethanol was distilled off under reduced pressure. The resultant aqueous layer was extracted three times with chloroform and the combined organic layer was dried over potassium carbonate. Distilling off the solvent under reduced pressure afforded crude 9-p-fluorobenzyl-3-oxa-9-azabicyclo[3.3.1]nonan-7-one oxime. This product was used for the subsequent reaction without any purification.

### Preparative Example 4

Synthesis of endo-7-amino-9-p-fluorobenzyl-3-oxa-9-azabicyclo[3.3.1]nonane

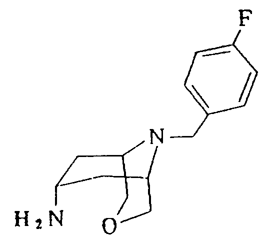

To a solution of 9-p-fluorobenzyl-3-oxa-9-azabicyclo[3.3.1]nonan-7-one oxime (13.0 g) in ethanol (300 ml) were added successively ammonium acetate (33.4 g) and an ethanol suspension of Raney nickel (20 ml) and stirred at 70°C in an autoclave in a hydrogen gas atmosphere at 50 atm for 7 hrs. To the reaction solution was added 10% aqueous sodium hydroxide solution (100 ml) and filtered through Celite. The filtrate was evaporated under reduced pressure to remove the solvent. The aqueous layer was extracted three times with ethyl acetate and the combined organic layer was dried over potassium carbonate. Distilling off the solvent under reduced pressure afforded crude endo-7-amino-9-p-fluorobenzyl-3-oxa-9-azabicyclo[3.3.1]nonane. This product was used for the subsequent reaction without any purification.

The following compounds were prepared in a similar manner.
endo-7-Amino-9-(m-fluorobenzyl)-3-oxa-9-azabicyclo[3.3.1]nonane
¹H NMR(CDCl₃) δ 1.40(d, J=16Hz, 2H), 1.86(bs, 2H), 2.42(td, J=7Hz, 16Hz, 2H), 2.62(bs, 2H), 3.23(t, J=7Hz, 1H), 3.70(d, J=11Hz, 2H), 3.80(s, 2H), 3.92(d, J=11Hz, 2H), 6.93(td, J=8Hz, 2Hz, 1H), 7.10-7.13(m, 2H), 7.23-7.29(m, 1H)
endo-7-Amino-9-(o-fluorobenzyl)-3-oxa-9-azabicyclo[3.3.1]nonane
¹H NMR(CDCl₃) δ 1.41(d, J=15Hz, 2H), 2.04(bs, 2H), 2.47(td, J=7Hz, 15Hz, 2H), 2.64(bs, 2H), 3.24(t, J=7Hz, 1H), 3.70(d, J=11Hz, 2H), 3.85(s, 2H), 3.92(d, J=11Hz, 2H), 7.01(t, J=9Hz, 1H), 7.11(t, J=7Hz, 1H), 7.18-7.25(m, 1H), 7.48(td, J=7Hz, 1Hz, 1H)
endo-7-Amino-9-(2,4-dichlorobenzyl)-3-oxa-9-azabicyclo[3.3.1]nonane
¹H NMR(CDCl₃) δ 1.44(d, J=15Hz, 2H), 2.17-2.40(b, 2H), 2.45(td, J=6Hz, 15Hz, 2H), 2.62(bs, 2H), 3.26(t, J=7Hz, 1H), 3.71(d, J=11Hz, 2H), 3.84(s, 2H), 3.93(d, J=11Hz, 2H), 7.22(dd, J=2Hz, 8Hz, 1H), 7.35(d, J=2Hz, 1H), 7.47(d, J=8Hz, 1H)
endo-7-Amino-9-(3,4-dichlorobenzyl)-3-oxa-9-azabicyclo[3.3.1]nonane
¹H NMR(CDCl₃) δ 1.40(d, J=15Hz, 2H), 2.04(bs, 2H), 2.40(td, J=7Hz, 15Hz, 2H), 2.61(bs, 2H), 3.22(t, J=7Hz, 1H), 3.70(d, J=11Hz, 2H), 3.75(s, 2H), 3.90(d, J=11Hz, 2H), 7.20(dd, J=1Hz, 8Hz, 1H), 7.37(d, J=8Hz, 1H), 7.47(d, J=1Hz, 1H)
endo-7-Amino-9-(p-chlorobenzyl)-3-oxa-9-azabicyclo[3.3.1]nonane
IR(neat) 3350, 2914, 2850, 1490, 1354, 1149, 1086, 847cm⁻¹
Endo-7-amino-9-(p-trifluoromethylbenzyl)-3-oxa-9-azabicyclo[3.3.1]nonane
¹H NMR(CDCl₃) δ 1.40(d, J=16Hz, 2H), 2.39-2.45(m, 4H), 2.62(d, J=4Hz, 2H), 3.24(td, J=6Hz, 2Hz, 1H), 3.71(d, J=11Hz, 2H), 3.86(s, 2H), 3.92(d, J=11Hz, 2H), 7.49(d, J=8Hz, 2H) 7.57(d, J=8Hz, 2H)
endo-7-Amino-9-benzyl-3-oxa-9-azabicyclo[3.3.1]nonane
Ms m/z 232(M⁺)
endo-7-Amino-9-(p-methoxybenzyl)-3-oxa-9-azabicyclo[3.3.1]nonan-7-one
¹H NMR(CDCl₃) δ 1.36(d, J=15Hz, 2H), 2.42(td, J=6Hz, 15Hz, 2H), 2.61(d, J=5Hz, 2H), 3.22(t, J=8Hz, 1H), 3.68(d, J=10Hz, 2H), 3.74(s, 2H), 3.80(s, 3H), 3.88(d, J=11Hz, 2H), 6.85(d, J=9Hz, 2H), 7.27(d, J=8Hz, 2H)

### Example 1

Synthesis of N-[endo-9-(p-fluorobenzyl)-3-oxa-9-azabicyclo[3.3.1]non-7-yl]-1H-indazole-3-carboxamide

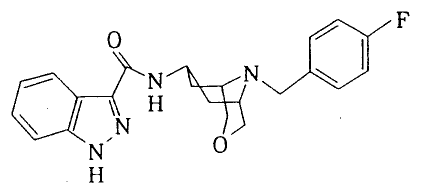

To a solution of endo-7-amino-9-(p-fluorobenzyl)-3-oxa-9-azabicyclo[3.3.1]nonane (2.03 g, 8.12 mmol) in DMF (50 ml) were added under ice-cooling potassium carbonate (1.68 g, 12.2 mmol), dimethylaminopyridine (0.60 g) and diindazolo[2,3-a][2',3'-d]pyrazine-7,14-dione (1.29 g). The mixture was stirred for 2.5 hrs. The reaction solution was added to water (300 ml) and extracted three times with ethyl acetate. The organic layer was washed with a saturated NaCl solution, dried over sodium sulfate and concentrated under reduced pressure. Recrystallization of the residue from ethanol afforded the title compound (2.34 g, 70% yield). m.p. 289-290°C
¹H NMR(CDCl₃) δ 1.56(d, J=15Hz, 2H), 2.47-2.54(m, 2H), 2.71(bs, 2H), 3.84(s, 2H), 3.88(d, J=11Hz, 2H), 4.04(d, J=11Hz, 2H), 4.82-4.88(m, 1H), 6.99-7.04(m, 2H), 7.26-7.43(m, 4H), 7.51(d, J=7Hz, 1H), 8.44(d, J=8Hz, 1H), 9.59(d, J=11Hz, 1H), 10.64(bs, 1H)
IR(KBr) 3148, 2918, 1642, 1548, 1507, 1218, 1149, 750cm⁻¹
Ms m/z 394(M⁺)

The following compounds were prepared in a similar manner.

### Example 2

N-[endo-9-(m-Fluorobenzyl)-3-oxa-9-azabicyclo[3.3.1]non-7-yl]-1H-indazole-3-carboxamide 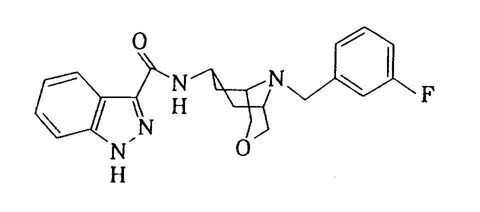
¹H NMR(DMSO-d₆) δ 1.42(d, J=15Hz, 2H), 2.40-2.60(m, 2H), 2.66(bs, 2H), 3.77(d, J=11Hz, 2H), 3.90(s, 2H), 3.92(d, J=11Hz, 2H), 4.60-4.67(m, 1H), 7.04(td, J=8Hz, 2Hz, 1H), 7.20-7.25(m, 3H), 7.33-7.42(m, 2H), 7.59(d, J=8Hz, 1H), 8.19(d, J=8Hz, 1H), 9.36(d, J=11Hz, 1H), 13.43(s, 1H)

### Example 3

N-[endo-9-(o-Fluorobenzyl)-3-oxa-9-azabicyclo[3.3.1]non-7-yl]-1H-indazole-3-carboxamide 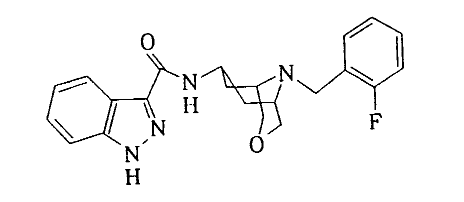
¹H NMR(DMSO-d₆) δ 1.45(d, J=15Hz, 2H), 2.45-2.60(m, 2H), 2.69(bs, 2H), 3.77(d, J=11Hz, 2H), 3.90(d, J=11Hz, 2H), 3.91(s, 2H), 4.61-4.68(m, 1H), 7.11-7.60(m, 7H), 8.20(d, J=8Hz, 1H), 9.37(d, J=11Hz, 1H), 13.42(bs, 1H)

### Example 4

N-[endo-9-(2,4-Dichlorobenzyl)-3-oxa-9-azabicyclo[3.3.1]non-7-yl]-1H-indazole-3-carboxamide 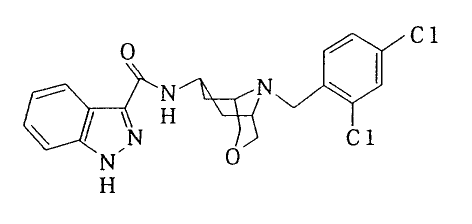
¹H NMR(CDCl₃) δ 1.62(d, J=15Hz, 2H), 2.55(td, J=6Hz, 15Hz, 2H), 2.72(bs, 2H), 3.89(d, J=11Hz, 2H), 3.92(s, 2H), 4.07(d, J=11Hz, 2H), 4.87(td, J=7Hz, 10Hz, 1H), 7.22-7.51(m, 6H), 8.44(d, J=8Hz, 1H), 9.57(d, J=11Hz, 1H), 10.32(bs, 1H)

### Example 5

N-[endo-9-(3,4-Dichlorobenzyl)-3-oxa-9-azabicyclo[3.3.1]non-7-yl]-1H-indazole-3-carboxamide 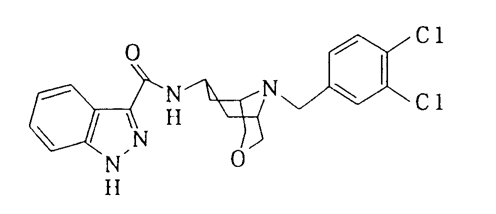
¹H NMR(DMSO-d₃) δ 1.47(d, J=15Hz, 2H), 2.44-2.53(m, 2H), 2.68(bs, 2H), 3.81(d, J=11Hz, 2H), 3.88(s, 2H), 3.96(d, J=11Hz, 2H), 4.50-4.70(m, 1H), 7.17-7.59(m, 6H), 8.21(d, J=8Hz, 1H), 9.39(d, J=11Hz, 1H), 13.32(bs, 1H)

### Example 6

N-[endo-9-(p-Chlorobenzyl)-3-oxa-9-azabicyclo[3.3.1]non-7-yl]-1H-indazole-3-carboxamide 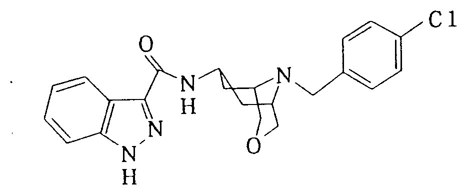
¹H NMR(CDCl₃) δ 1.56(d, J=15Hz, 2H), 2.50(td, J=6Hz, 14Hz, 2H), 2.71(bs, 2H), 3.85(s, 2H), 3.87(d, J=11Hz, 2H), 4.04(d, J=11Hz, 2H), 4.84(td, J=7Hz, 10Hz, 1H), 7.26-7.35(m, 5H), 7.40-7.44(m, 1H), 7.50(d, J=8Hz, 1H), 8.44(d, J=8Hz, 1H), 9.57(d, J=11Hz, 1H), 10.45(s, 1H)

### Example 7

N-[endo-9-(p-Trifluoromethylbenzyl)-3-oxa-9-azabicyclo[3.3.1]non-7-yl]-1H-indazole-3-carboxamide 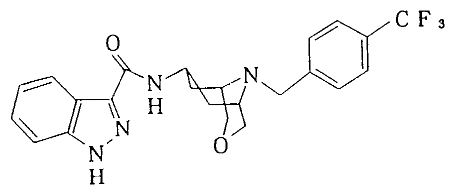
¹H NMR(CDCl₃) δ 1.58(d, J=15Hz, 2H), 2.52(td, J=6Hz, 15Hz, 2H), 2.72(bs, 2H), 3.90(d, J=11Hz, 2H), 3.94(s, 2H), 4.07(d, J=11Hz, 2H), 4.86(td, J=7Hz, 10Hz, 1H), 7.29(t, J=7Hz, 1H), 7.41(t, J=7Hz, 1H), 7.49-7.60(m, 5H), 8.44(d, J=8Hz, 1H), 9.57(d, J=11Hz, 1H), 10.22(bs, 1H)

### Example 8

N-[endo-9-Benzyl-3-oxa-9-azabicyclo[3.3.1]non-7-yl]-1H-indazole-3-carboxamide 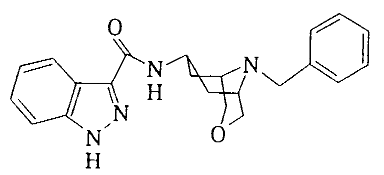
¹H NMR(CDCl₃) δ 1.56(d, J=15Hz, 2H), 2.54(td, J=6Hz, 15Hz, 2H), 2.74(bs, 2H), 3.88(d, J=11Hz, 2H), 3.90(s, 2H), 4.07(d, J=11Hz, 2H), 4.85(td, J=7Hz, 11Hz, 1H), 7.24-7.51(m, 7H), 8.44(d, J=8Hz, 1H), 9.62(d, J=11Hz, 1H), 10.28(bs, 1H)

### Example 9

N-[endo-9-(p-Methoxybenzyl)-3-oxa-9-azabicyclo[3.3.1]non-7-yl]-1H-indazole-3-carboxamide 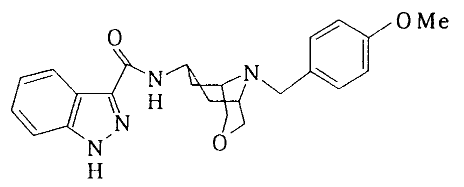
¹H NMR(CDCl₃) δ 1.54(d, J=15Hz, 2H), 2.52(td, J=6Hz, 15Hz, 2H), 2.73(bs, 2H), 3.80(s, 3H), 3.82(s, 2H), 3.88(d, J=11Hz, 2H), 4.05(d, J=10Hz, 2H), 4.85(td, J=7Hz, 10Hz, 1H), 6.87(d, J=9Hz, 2H), 7.29-7.32(m, 3H), 7.42(td, J=8Hz, 1Hz, 1H), 7.50(d, J=8Hz, 1H), 8.44(d, J=8Hz, 1H), 9.61(d, J=11Hz, 1H), 10.54(bs, 1H)

### Example 10

Synthesis of N-[endo-9-(p-fluorobenzyl)-3-oxa-9-azabicyclo[3.3.1]non-7-yl]-1-(3-methyl-2-butenyl)indazole-3-carboxamide 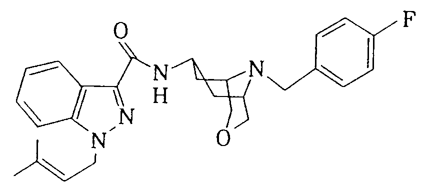
N-[endo-9-(p-Fluorobenzyl)-3-oxa-9-azabicyclo[3.3.1]non-7-yl]-1H-indazole-3-carboxamide (2.34 g) was dissolved in DMF (50 ml) and to the solution was added 60% sodium hydride (0.28 g) under ice-cooling. After stirring the solution under ice-cooling for 20 minutes and at room temperature for one hour, 1-bromo-3-methyl-2-butene (1.06 g) was added and stirred for 3.5 hrs. The reaction solution was added to water (300 ml) and extracted three times with ethyl acetate. The organic layer was washed with saturated NaCl solution, dried over sodium sulfate and concentrated under reduced pressure. Purification of the residue by silica gel column chromatography (2/1 hexane/ethyl acetate) afforded the title compound (1.47 g).

To the solution of the title compound (1.37 g) in ethyl acetate (15 ml) was added 4N hydrochloric acid/ethyl acetate solution (1 ml) under ice-cooling. The precipitated crystals was collected by filtration and dried under reduced pressure to give the hydrochloride (1.30 g).
m.p. 112-113°C(free) 198-201°C(hydrochloride)
¹H NMR(CDCl₃) δ 1.55(d, J=15Hz, 2H), 1.75(s, 3H), 1.88(s, 3H), 2.46-2.53(m, 2H), 2.69(bs, 2H), 3.84(s, 2H), 3.85(d, J=10Hz, 2H), 4.01(d, J=11Hz, 2H), 4.84(td, J=7Hz, 11Hz, 1H), 5.01(d, J=7Hz, 2H), 5.40-5.44(m, 1H), 6.98-7.04(m, 2H), 7.22-7.26(m, 1H), 7.34-7.38(m, 4H), 8.37(d, J=8Hz, 1H), 9.37(d, J=11Hz, 1H)
IR(KBr) 3324, 2912, 1663, 1532, 1509, 1257, 1217, 1149, 848, 746cm⁻¹

The following compounds were prepared in a similar manner.

### Example 11

N-[endo-9-(p-Fluorobenzyl)-3-oxa-9-azabicyclo[3.3.1]non-7-yl]-1-(n-propyl)indazole-3-carboxamide 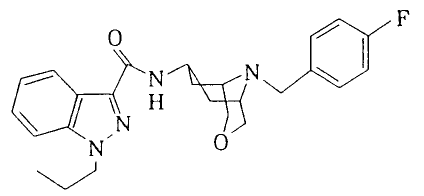
¹H NMR(CDCl₃) δ 0.94(t, J=7Hz, 3H), 1.55(d, J=15Hz, 2H), 1.97(sext, J=7Hz, 2H), 2.46-2.54(m, 2H), 2.69(bs, 2H), 3.84(s, 2H), 3.85(d, J=11Hz, 2H), 4.02(d, J=11Hz, 2H), 4.35(t, J=7Hz, 2H), 4.81-4.88(m, 1H), 7.02(t, J=9Hz, 2H), 7.23-7.28(m, 1H), 7.34-7.42(m, 4H), 8.39(d, J=8Hz, 1H), 9.39(d, J=11Hz, 1H)

### Example 12

N-[endo-9-(p-Fluorobenzyl)-3-oxa-9-azabicyclo[3.3.1]non-7-yl]-1-(isopropyl)indazole-3-carboxamide 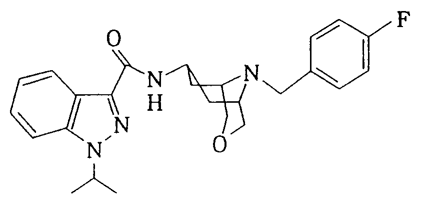
¹H NMR(CDCl₃) δ 1.55(d, J=15Hz, 2H), 1.60(d, J=7Hz, 6H), 2.46-2.54(m, 2H), 2.69(bs, 2H), 3.84(d, J=11Hz, 2H), 3.85(s, 2H), 4.02(d, J=11Hz, 2H), 4.80-4.92(m, 2H), 7.02(t, J=9Hz, 2H), 7.21-7.26(m, 1H), 7.34-7.40(m, 3H), 7.45(d, J=9Hz), 8.39(d, J=8Hz, 1H), 9.51(d, J=11Hz, 1H)

### Example 13

N-[endo-9-(p-Fluorobenzyl)-3-oxa-9-azabicyclo[3.3.1]non-7-yl]-1-(isobutyl)indazole-3-carboxamide 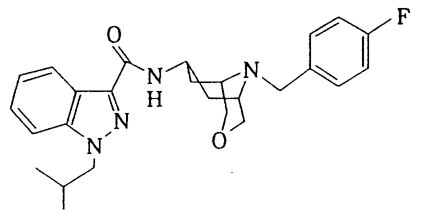
¹H NMR(CDCl₃) δ 0.93(d, J=7Hz, 6H), 1.55(d, J=15Hz, 2H), 2.34(sep, J=7Hz, 1H), 2.46-2.54(m, 2H), 2.69(bs, 2H), 3.84(s, 2H), 3.84(d, J=11Hz, 2H), 4.01(d, J=11Hz, 2H), 4.19(d, J=7Hz, 2H), 4.81-4.88(m, 1H), 7.02(t, J=9Hz, 2H), 7.22-7.26(m, 1H), 7.34-7.40(m, 4H), 8.38(d, J=8Hz, 1H), 9.40(d, J=11Hz, 1H)

### Example 14

N-[endo-9-(p-Fluorobenzyl)-3-oxa-9-azabicyclo[3.3.1]non-7-yl]-1-allylindazole-3-carboxamide 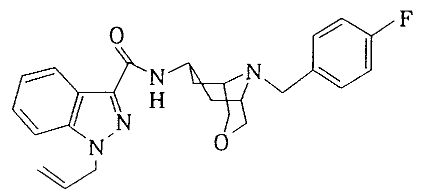
¹H NMR(CDCl₃) δ 1.55(d, J=15Hz, 2H), 2.50(td, J=7Hz, 15Hz, 2H), 2.69(bs, 2H), 3.87(s, 2H), 3.94(d, J=11Hz, 2H), 4.01(d, J=11Hz, 2H), 4.85(td, J=7Hz, 11Hz, 1H), 5.05(d, J=6Hz, 2H), 5.16(d, J=17Hz, 1H), 5.24(d, J=10Hz, 1H), 5.98-6.08(m, 1H), 7.02(t, J=8Hz, 2H), 7.24-7.29(m, 2H), 7.34-7.40(m, 3H), 8.39(d, J=8Hz, 1H), 9.36(d, J=10Hz, 1H)

### Example 15

N-[endo-9-(p-Fluorobenzyl)-3-oxa-9-azabicyclo[3.3.1]non-7-yl]-1-(cyclopropylmethyl)indazole-3-carboxamide 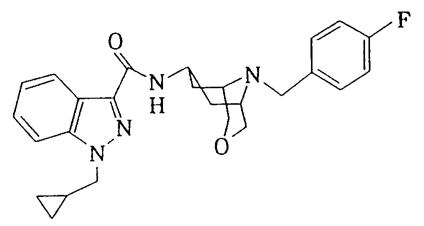
m.p. 125-127°C(free) 205-207°C(hydrochloride)

### Example 16

N-[endo-9-Benzyl-3-oxa-9-azabicyclo[3.3.1]non-7-yl]-1-allylindazole-3-carboxamide 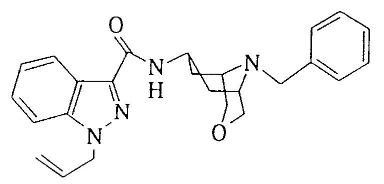
¹H NMR(CDCl₃) δ 1.55(d, J=15Hz, 21H), 2.53(td, J=6Hz, 15Hz, 2H), 2.72(bs, 2H), 3.86(d, J=11Hz, 2H), 3.89(s, 2H), 4.04(d, J=11Hz, 2H), 4.85(td, J=7Hz, 10Hz, 1H), 5.05(d, J=6Hz, 2H), 5.16(d, J=18Hz, 1H), 5.24(d, J=11Hz, 1H), 5.99-6.08(m, 1H), 7.24-7.41(m, 8H), 8.40(d, J=8Hz, 1H), 9.38(d, J=11Hz, 1H)

### Example 17

N-[endo-9-Benzyl-3-oxa-9-azabicyclo[3.3.1]non-7-yl]-1-(cyclopropylmethyl)indazole-3-carboxamide 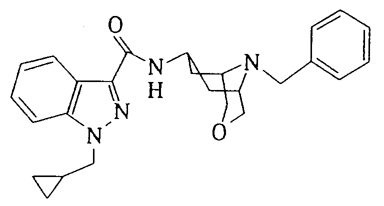
¹H NMR(CDCl₃) δ 0.43-0.47(m, 2H), 0.56-0.61(m, 2H), 1.31-1.39(m, 1H), 1.55(d, J=15Hz, 2H), 2.53(td, J=6Hz, 15Hz, 2H), 2.72(bs, 2H), 3.85(d, J=11Hz, 2H), 3.89(s, 2H), 4.04(d, J=11Hz, 2H), 4.28(d, J=7Hz, 2H), 4.85(td, J=7Hz, 10Hz, 1H), 7.24-7.52(m, 8H), 8.40(d, J=8Hz, 1H), 9.47(d, J=11Hz, 1H)

### Example 18

N-[endo-9-(o-Fluorobenzyl)-3-oxa-9-azabicyclo[3.3.1]non-7-yl]-1-(n-propyl)indazole-3-carboxamide 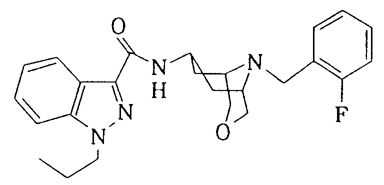
¹H NMR(CDCl₃) δ 0.94(t, J=8Hz, 3H), 1.58(d, J=15Hz, 2H), 1.97(sext, J=8Hz, 2H), 2.52-2.60(m, 2H), 2.73(bs, 2H), 3.85(d, J=11Hz, 2H), 3.92(s, 2H), 4.05(d, J=11Hz, 2H), 4.35(t, J=8Hz, 2H), 4.82-4.88(m, 1H), 7.03(t, J=9Hz, 1H), 7.13(t, J=7Hz, 1H), 7.20-7.42(m, 4H), 7.49(t, J=7Hz, 1H), 8.39(d, J=8Hz, 1H), 9.39(d, J=10Hz, 1H)

### Example 19

N-[endo-9-(m-Fluorobenzyl)-3-oxa-9-azabicyclo[3.3.1]non-7-yl]-1-allylindazole-3-carboxamide 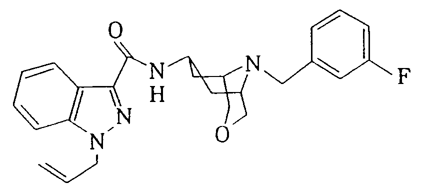
¹H NMR(CDCl₃) δ 1.50-1.60(m, 2H), 2.46-2.55(m, 2H), 2.71(bs, 2H), 3.87(d, J=11Hz, 2H), 3.88(s, 2H), 4.04(d, J=11Hz, 2H), 4.81-4.89(m, 1H), 5.05(d, J=5Hz, 2H), 5.17(d, J=17Hz, 1H), 5.24(d, J=10Hz, 1H), 5.98-6.08(m, 1H), 6.95(td, J=8Hz, 2Hz, 1H), 7.14-7.25(m, 2H), 7.26-7.39(m, 4H), 8.40(d, J=8Hz, 1H), 9.36(d, J=11Hz, 1H)

### Example 20

N-[endo-9-(p-Methoxybenzyl)-3-oxa-9-azabicyclo[3.3.1]non-7-yl]-1-ethylindazole-3-carboxamide 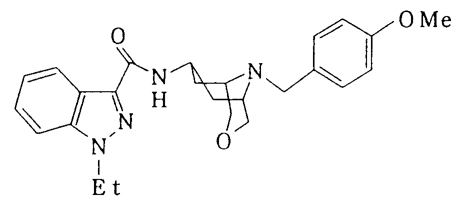
¹H NMR(CDCl₃) δ 1.53(t, J=7Hz, 3H), 1.56(d, J=15Hz, 2H), 2.51 (td, J=5Hz, 15Hz, 2H), 2.71(bs, 2H), 3.81(s, 3H), 3.82(s, 2H), 3.85(d, J=11Hz, 2H), 4.02(d, J=11Hz, 2H), 4.45(q, J=7Hz, 2H), 4.84 (td, J=7Hz, 11Hz, 1H), 6.87(d, J=9Hz, 2H), 7.23-7.27(m, 1H), 7.31(d, J=9Hz, 2H), 7.36-7.42(m, 2H), 8.39(d, J=8Hz, 1H), 9.40(d, J=11Hz, 1H)

### Example 21

N-[endo-9-(p-Methoxybenzyl)-3-oxa-9-azabicyclo[3.3.1]non-7-yl]-1-(n-propyl)indazole-3-carboxamide 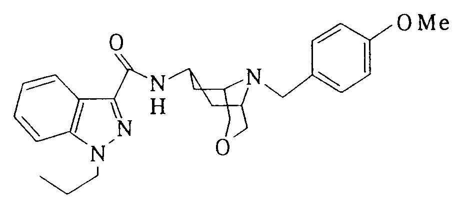
¹H NMR(CDCl₃) δ 0.94(t, J=7Hz, 3H), 1.53(d, J=15Hz, 2H), 1.96 (sext, J=7Hz, 2H), 2.51(td, J=6Hz, 15Hz, 2H), 2.70(bs, 2H), 3.81 (s, 3H), 3.82(s, 2H), 3.84(d, J=11Hz, 2H), 4.01(d, J=11Hz, 2H), 4.35(t, J=7Hz, 2H), 4.84(td, J=7Hz, 11Hz, 1H), 6.87(d, J=9Hz, 2H), 7.25-7.27(m, 1H), 7.31(d, J=8Hz, 2H), 7.35-7.42(m, 2H), 8.38(d, J=8Hz, 1H); 9.40(d, J=11Hz, 1H)

### Example 22

N-[endo-9-(p-Methoxybenzyl)-3-oxa-9-azabicyclo[3.3.1]non-7-yl]-1-(isopropyl)indazole-3-carboxamide 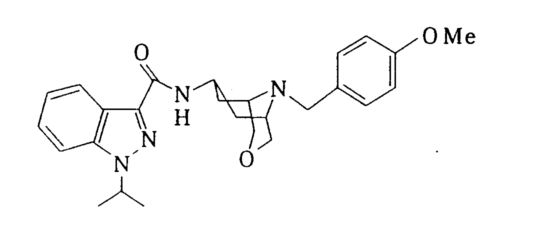
¹H NMR(CDCl₃) δ 1.53(d, J=15Hz, 2H), 1.60(d, J=6Hz, 6H), 2.50 (td, J=6Hz, 15Hz, 2H), 2.70(bs, 2H), 3.80(s, 3H), 3.85(s, 2H), 3.83(d, J=10Hz, 2H), 4.01(d, J=11Hz, 2H), 4.80-4.90(m, 2H), 6.87 (d, J=8Hz, 2H), 7.22-7.26(m, 1H), 7.32(d, J=9Hz, 2H), 7.37(t, J=8Hz, 1H), 7.44(d, J=8Hz 1H), 8.38(d, J=8Hz, 1H), 9.52(d, J=11Hz, 1H)

### Example 23

N-[endo-9-(p-Methoxybenzyl)-3-oxa-9-azabicyclo[3.3.1]non-7-yl]-1-(n-butyl)indazole-3-carboxamide 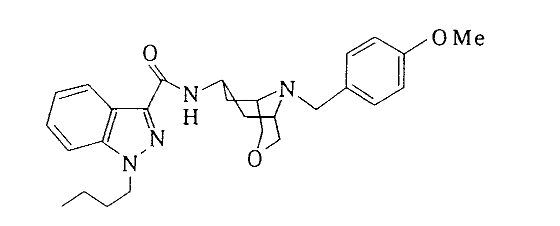
¹H NMR(CDCl₃) δ 0,94(t, J=7Hz, 3H), 1.34(sext, J=7Hz, 2H), 1.53(d, J=15Hz, 2H), 1.91(quint, J=7Hz, 2H), 2.50(td, J=6Hz, 15Hz, 2H), 2.70(bs, 2H), 3.80(s, 3H), 3.82(s, 2H), 3.84(d, J=13Hz, 2H), 4.02(d, J=11Hz, 2H), 4.39(t, J=7Hz, 2H), 4.83(td, J=7Hz, 11Hz, 1H), 6.87(d, J=9Hz, 2H), 7.23-7.27(m, 1H), 7.31(d, J=9Hz, 2H), 7.36-7.41(m, 2H), 8.38(d, J=8Hz, 1H), 9.40(d, J=11Hz, 1H)

### Example 24

N-[endo-9-(p-Methoxybenzyl)-3-oxa-9-azabicyclo[3.3.1]non-7-yl]-1-(isobutyl)indazole-3-carboxamide 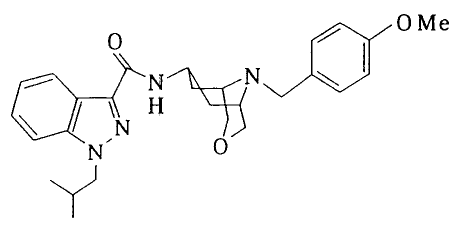
¹H NMR(CDCl₃) δ 0.93(d, J=7H, 6H), 1.53(d, J=15Hz, 2H), 2.34 (sept, J=7Hz, 1H), 2.51(td, J=7Hz, 15Hz, 2H), 2.70(bs, 2H), 3.80 (s, 3H), 3.82(s, 2H), 3.83(d, J=10Hz, 2H), 4.01(d, J=11Hz, 2H), 4.18(d, J=7Hz, 2H), 4.84(td, J=7Hz, 10Hz, 1H), 6.87(d, J=8Hz, 2H), 7.22-7.28(m, 1H), 7.31(d, J=9Hz, 2H), 7.35-7.40(m, 2H), 8.39(d, J=8Hz, 1H), 9.41(d, J=11Hz, 1H)

### Example 25

N-[endo-9-(p-Methoxybenzyl)-3-oxa-9-azabicyclo[3.3.1]non-7-yl]-1-allylindazole-3-carboxamide 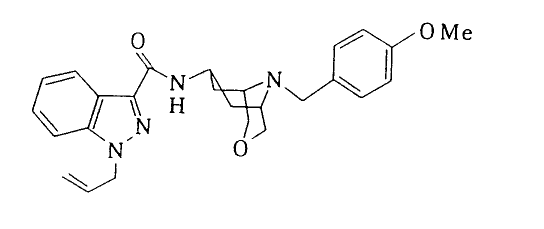
¹H NMR(CDCl₃) δ 1.53(d, J=15Hz, 2H), 2.51(td, J=6Hz, 15Hz, 2H), 2.76(bs, 2H), 3.80(s, 3H), 3.82(s, 2H), 3.84(d, J=11Hz, 2H), 4.01(d, J=11Hz, 2H), 4.84(td, J=7Hz, 10Hz, 1H), 5.04(dd, J=1Hz, 15Hz, 2H), 5.16(d, J=17Hz, 1H), 5.24(dd, J=1Hz, 10Hz, 1H), 5.98-6.06(m, 1H), 6.87(d, J=8Hz, 2H), 7.25(d, J=1Hz, 1H), 7.33(d, J=8Hz, 2H), 7.35-7.40(m, 2H), 8.39(dd, J=1Hz, 8Hz, 1H), 9.37(d, J=11Hz, 1H)

### Example 26

N-[endo-9-(p-Methoxybenzyl)-3-oxa-9-azabicyclo[3.3.1]non-7-yl]-1-(3-methyl-2-butenyl)indazole-3-carboxamide 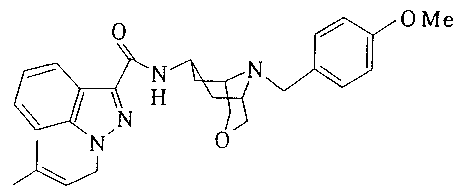
¹H NMR(CDCl₃) δ 1.53(d, J=14Hz, 2H), 1.74(s, 3H), 1.88(s, 3H), 2.55(td, J=6Hz, 14Hz, 2H), 2.70(bs, 2H), 3.80(s, 3H), 3.82(s, 2H), 3.84(d, J=11Hz, 2H), 4.00(d, J=11Hz, 2H), 4.83(td, J=7Hz, 18Hz, 1H), 5.01(d, J=7Hz, 2H), 5.40-5.43(m, 1H), 6.87(d, J=9Hz, 2H), 7, 22-7.28(m, 1H), 7.31(d, J=8Hz, 2H), 7.36-7.37(m, 2H), 8.38(d, J=8Hz, 1H), 9.39(d, J=11Hz, 1H)

### Example 27

N-[endo-9-(p-Methoxybenzyl)-3-oxa-9-azabicyclo[3.3.1]non-7-yl]-1-(cyclopropylmethyl)indazole-3-carboxamide 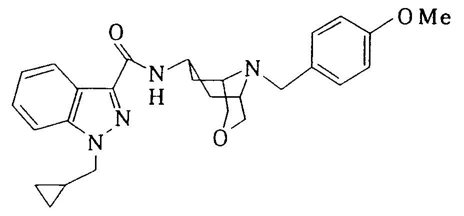
¹H NMR(CDCl₃) δ 0.45(d, J=6Hz, 2H), 0.58(d, J=6Hz, 2H), 1.31-1.35(m, 1H), 1.53(d, J=14Hz, 2H), 2.51(td, J=7Hz, 15Hz, 2H), 2.71(bs, 2H), 3.81(s, 3H), 3.82(s, 2H), 3.84(d, J=14Hz, 2H), 4.01(d, J=11Hz, 2H), 4.27(d, J=7Hz, 2H), 4.84(td, J=7Hz, 10Hz, 1H), 6.87(d, J=8Hz, 2H), 7, 23-7.27(m, 1H), 7.31(d, J=8Hz, 2H), 7.36-7.52(m, 2H), 8.39(d, J=8Hz, 1H), 9.46(d, J=11Hz, 1H)

### Example 28

Synthesis of N-[endo-9-(3, 4-dichlorobenzyl)-3-oxa-9-azabicyclo[3.3.1]non-7-yl]-1-allylindazole-3-carboxamide and N-[endo-9-(3,4-dichlorobenzyl)-3-oxa-9-azabicyclo[3.3.1]non-7-yl]-1-(1-propenyl)indazole-3-carboxamide

To a solution of N-[endo-9-(3,4-dichlorobenzyl)-3-oxa-9-azabicyclo[3.3.1]non-7-yl]-1H-indazole-3-carboxamide (1.13 g, 2.53 mmol) in DMF (10 ml) was added 60% sodium hydride (0.12 g, 3.00 mmol) and stirred at room temperature for 3 hrs. Allyl bromide (0.37 g, 3.06 mol) was added and further stirring was continued for 3 hrs. After allowing to stand overnight, DMF was distilled off and the residue was extracted with ethyl acetate. The organic layer was dried over anhydrous magnesium sulfate and distilled off. Purification of the residue by silica gel chromatography (hexane-ethyl acetate = 3/1 - 0/1) gave a fraction of the 1-propenyl form and the allyl form. The allyl form was obtained as crystals (0.53 g, 43% yield) in ether/hexane. The conversion of both forms to the hydrochloride by conventional method afforded the allyl and 1-propenyl forms (0.38 g, 29% yield) as crystals.
N-[endo-9-(3,4-Dichlorobenzyl)-3-oxa-9-azabicyclo[3.3.1]non-7-yl]-1-allylindazole-3-carboxamide 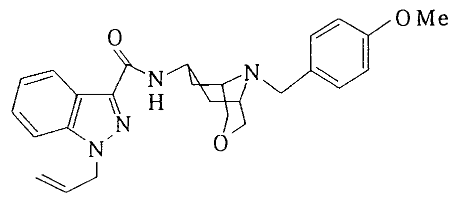
¹H NMR(CDCl₃) δ 1.57(d, J=15Hz, 2H), 2.44-2.52(m, 2H), 2.68(bs, 2H), 3.83(s, 2H), 3.87(d, J=11Hz, 2H), 4.02(d, J=11Hz, 2H), 4.81-4.88(m, 1H), 5.05(d, J=5Hz, 2H), 5.17(dd, J=1Hz, 17Hz, 1H), 5.24(d, J=10Hz, 1H), 5.98-6.09(m, 1H), 7.22-7.40(m, 5H), 7.51(d, J=1Hz, 1H), 8.39(d, J=8Hz, 1H), 9.34(d, J=11Hz, 1H)
N-[endo-9-(3,4-Dichlorobenzyl)-3-oxa-9-azabicyclo[3.3.1]non-7-yl]-1-(1-propenyl)indazole-3-carboxamide 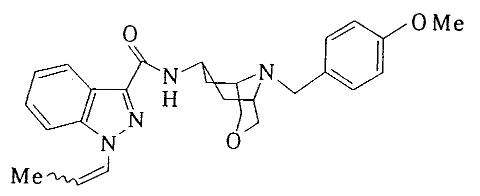
¹H NMR(CDCl₃) δ 1.55(d, J=15Hz, 2H), 1.95(dd, J=2HZ, 7Hz, 0.9H), 2.09(dd, J=2Hz, 7Hz, 2.1H), 2.44-2.53(m, 2H), 2.69(bs, 2H), 3.83(s, 2H), 3.83-4.05(m, 4H), 4.80-4.87(m, 1H), 5.58(qd, J=7Hz, 9Hz, 0.7H), 6.38(qd, J=7Hz, 14Hz, 0.3H), 6.97(dd, J=2Hz, 9Hz, 0.7H), 7.12(dd, J=2Hz, 14Hz, 0.3H), 7.22-7.52(m, 6H), 8.40(d, J=8Hz, 0.3H), 8.41(d, J=8Hz, 0.7H), 9.53(d, J=11Hz, 0.3H), 9.61(d, J=11Hz, 0.7H)

### Example 29

Synthesis of N-[endo-9-(p-fluorobenzyl)-3-oxa-9-azabicyclo[3.3.1]non-7-yl]-4-amino-5-chloro-2-methoxybenzamide

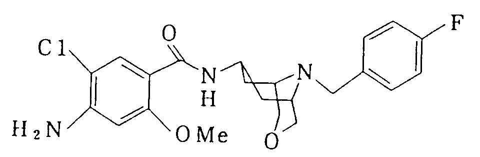

To a solution of endo-7-amino-9-(p-fluorobenzyl)-3-oxa-9-azabicyclo[3.3.1]nonane (0.66 g) and 4-amino-5-chloro-2-methoxybenzoic acid (0.53 g) in DMF (20 ml) was added under ice-cooling 1-hydroxybenzotriazole (0.39 g) and stirred for 30 minutes. 1-Ethyl-3-(3'-dimethylaminopropyl)carbodiimide hydrochloride (0.56 g) was added and stirring was continued under ice-cooling for one hour and at room temprature for 9 hrs. The reaction solution in water (150 ml) was extracted three times with ethyl acetate. The organic layer was washed with saturated NaCl solution, dried over sodium sulfate and concentrated under reduced pressure. Recrystallization of the residue from ethyl acetate afforded the title compound (0.56 g).

To a solution of the title compound (0.51 g) in a mixed solution of chloroform (20 ml) and methanol was added under ice-cooling 4N hydrochloric acid/ethyl acetate solution (1 ml). The precipitated crystals was collected by filtration and dried under reduced pressure to give the hydrochloride (0.36 g).
m.p. 253-255°C(free) 250-252°C(hydrochloride)
¹H NMR(CDCl₃) δ 1.44(d, J=15Hz, 2H), 2.41-2.48(m, 2H), 2.64(bs, 2H), 3.75(d, J=11Hz, 2H), 3.81(s, 3H), 3.84(s, 3H), 3.95(d, J=11Hz, 2H), 4.31(s, 2H), 4.82-4.89(m, 1H), 6.26(s, 1H), 6.99-7.03(m, 2H), 7.33-7.37(m, 1H), 8.10(s, 1H), 9.63(d, J=10Hz, 1H)
IR(KBr) 3312, 1628, 1599, 1491, 1247, 1218, 1115, 859cm⁻¹

### Example 30

The gastrointestinal motility promoting action of the present compounds was tested by the following method.

Ileum was excised from guinea pigs to prepare longitudinal muscle with adherent myenteric plexus preparation (approximately 2 cm length). The preparation suspended in organ bath containing Krebs-Henselite solution aerated with a mixed gas (5% CO₂, 95% O₂) were subjected to transmural electrical stimulation (1 msec, 0.1 Hz). Consistent twitch response induced by electrical stimulation and increase of twitch response induced by compound were recorded with an isometric transducer. The compounds were dissolved in distilled water or DMSO and tested at concentration of 10⁻⁵ M. The result is shown in terms of percent increase (%) in the contraction height by the electrical stimulation after administration of the compound relative to the contraction height before administration of the compound.
- Compound of Example 10: 16.1%
- Compound of Example 29: 22.4%

Whether or not the action of the above compounds is that via activation of 5-HT₄ receptors was investigated using as 5-HT₄ receptor antagonist, SDZ 205-557 (2-methoxy-4-amino-5-chloro-benzoic acid 2-(diethylamino)ethyl ester) described in Naunyn-Schmiedeberg's Arch Pharmacol (1992) 345:387-393. Thus, the investigation as to whether SDZ 205-557 can antagonize the increase in the contraction height of the above compounds using the longitudinal muscle preparation of the isolated guinea pig ileum can reveal that such increase in the contraction height is the action via activation of 5-HT₄ receptors.

It was verified that the increase in the contraction height of the above compounds was antagonized with 3x10⁻⁷ M SDZ 205-557 and this increase was the effect via 5-HT₄ receptors.

### Example 31

The activity of oxazabicyclo derivatives and Mosapride on 5-HT₄ receptors was determined by the procedure mentioned in Naunyn-Schmiedeberg's Arch Pharmacol (1991) 343:439-446.

The rat oesophageal tunica muscularis mucosae was suspended in organ both containing Krebs-Henseleit solution aerated with a mixed gas (95% O₂, 5% CO₂) and contracted with carbachol (1x10⁻⁶ M). After the contraction was stabilized, the cumulative administration of the compounds was performed to determine the relaxation of the rat oesophagus pre-contracted with carbachol. The concentration (ED₅₀) to cause 50% relaxation of the carbachol-induced contraction was measured. The result is expressed in terms of -log ED₅₀ and shown in the following table in which higher numerical value indicates higher activity.

| Compound of Example No. | Activity (-log ED₅₀) |
|---|---|
| 11 | 4.41 |
| 12 | 4.74 |
| 13 | 4.20 |
| 14 | 4.80 |
| 15 | 4.18 |
| 16 | 5.28 |
| 17 | 4.70 |
| 18 | 4.31 |
| 20 | 5.33 |
| 21 | 4.88 |
| 22 | 4.83 |
| 23 | 3.87 |
| 24 | 4.35 |
| 25 | 5.03 |
| 26 | 3.52 |
| 27 | 4.55 |
| Mosapride | 4.46 |

The following examples illustrate pharmaceutical formulations.

| Tablets (per tablet) | |
|---|---|
| Compound of Example 1 (Active ingredient) | 10 mg |
| Lactose | 67 mg |
| Crystalline cellulose | 15 mg |
| Corn starch | 7 mg |
| Magnesium stearate | 1 mg |

The above ingredients were uniformly blended to prepare powders for direct compression. The powders were formed in a rotary tableting machine to tablets each 6 mm in diameter and weighing 100 mg.

| Granules (per divided packet) | |
|---|---|
| Compound of Example 1 (Active ingredient) | 10 mg |
| Lactose | 90 mg |
| Corn starch | 50 mg |
| Crystalline cellulose | 50 mg |
| Hydroxypropylcellulose | 10 mg |
| Ethanol | 9 mg |

The active ingredient, lactose, corn starch and crystalline cellulose were uniformly blended and a solution of hydroxypropylcellulose in ethanol was added. The mixture was kneaded and granulated by an extrusion granulation method. The granules were then dried in a drier at 50°C. The dried granules were screened to granule sizes between 297 µm and 1460 µm to give a granule formulation weighing 200 mg per divided packet.

| Syrups Granules | |
|---|---|
| Compound of Example 2 (Active ingredient) | 1.000 g |
| Refined sugar | 30.000 g |
| D-sorbitol, 70 w/v% | 25.000 g |
| Ethyl paraoxybenzoate | 0.030 g |
| Propyl paraoxybenzoate | 0.015 g |
| Flavor | 0.200 g |
| Glycerin | 0.150 g |
| 96% Ethanol | 0.500 g |
| Distilled water | q.s. |

The active ingredient, refined sugar, D-sorbitol, ethyl paraoxybenzoate and propyl paraoxybenzoate were dissolved in 60 g of warm water. After cooling, glycerin and a solution of the flavor in ethanol were added. Distilled water was added to the mixture to make up a total amount of 100 ml.

| Injections | |
|---|---|
| Compound of Example 2 (Active ingredient) | 1 mg |
| Sodium chloride | 10 mg |
| Distilled water | q.s. |

The active ingredient and sodium chloride were dissolved in distilled water to make up a total amount of 1.0 ml.

| Suppositories per piece | |
|---|---|
| Compound of Example 1 (Active ingredient) | 2 g |
| Polyethylene glycol 4000 | 20 g |
| Glycerin | 78 g |

The active ingredient was dissolved in glycerin. To the solution was added polyethylene glycol 4000 and the mixture was warmed to a solution. The solution was poured into a suppository mold and solidified by cooling to prepare suppositories weighing 1.5 g per piece.

## Claims

1. A compound of formula (I) or a pharmaceutically acceptable salt thereof: 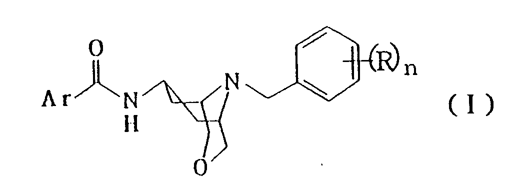 wherein R is a hydrogen atom, a halogen atom, a halo(C₁-C₆)alkyl group, a (C₁-C₆)alkoxy group, a nitro group, a hydroxyl group or an amino group, n is 1 or 2, the R groups being the same or different when n is 2, and Ar represents a radical of formula (II), (III), (IV), (V), (VI), (VII) or (VIII) 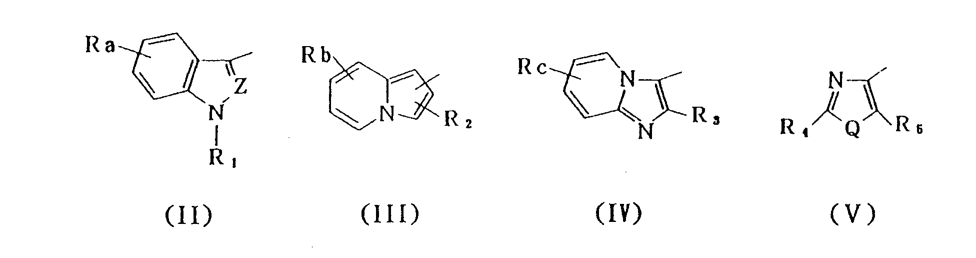 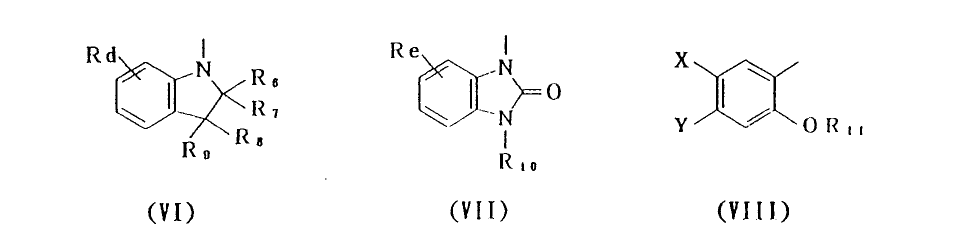 wherein
Ra to Re are independently a hydrogen atom, a halogen atom, a hydroxyl group, a (C₁-C₆)alkoxy group or a (C₁-C₈)alkyl group;
R₁ is a hydrogen atom, a (C₁-C₈)alkyl group, a (C₃-C₈)alkenyl group, a (C₃-C₈)alkynyl group, a (C₃-C₆)cycloalkyl group, a (C₃-C₆)cycloalkyl(C₁-C₆)alkyl group, a (C₁-C₆)alkoxy(C₂-C₆)alkyl group, a (C₃-C₆)oxoalkyl group, a (C₁-C₆)alkoxycarbonyl(C₁-C₆)alkyl group, a (C₁-C₆)alkoxycarbonyl group, a (C₁-C₆)alkanoyl group, a di(C₁-C₆)alkylamino(C₂-C₆)alkyl group, a hydroxy(C₂-C₆)alkyl group, a halo(C₁-C₆)alkyl group, a cyano(C₁-C₆)alkyl group, 4,6-diamino-2-triazinylmethyl group or a benzyl group optionally substituted by one or two substituents selected from the group consisting of halogen, C₁-C₆ alkoxy, nitro, hydroxyl and amino;
Z is CH or N;
R₂, R₃, R₅, R₆, R₉, R₁₀ and R₁₁ are independently a hydrogen atom or a (C₁-C₆)alkyl group;
R₄ is a (C₁-C₆)alkyl group, a pyridyl group or a phenyl group optionally substituted by halogen, C₁-C₆ alkyl, C₁-C₆ alkoxy or trifluoromethyl;
Q is N, S or O;
X is a halogen atom;
Y is NH₂ or a phthalimido group;
R₇ is a hydrogen atom;
R₈ is a hydrogen atom or a (C₁-C₄)alkyl group; or R₇ and R₈ together form a single bond.

2. A compound of claim 1 wherein Ar represents formula (II).

3. A compound of claim 2 wherein R is a halogen atom, a halo(C₁-C₄)alkyl group or a (C₁-C₄)alkoxy group, n is 1 or 2, Z is N, R₁ is a hydrogen atom, a (C₁-C₆)alkyl group, a (C₃-C₆)alkenyl group, a (C₃-C₆)alkynyl group, a (C₃-C₆)cycloalkyl group or a (C₃-C₆)cycloalkyl(C₁-C₆)alkyl group and Ra is a hydrogen atom.

4. A compound of claim 3 wherein Z is N.

5. A compound of claim 1 wherein Ar represents formula (VIII).

6. A compound of claim 5 wherein R is a halogen atom, a halo(C₁-C₄)alkyl group or a (C₁-C₆)alkoxy group, n is 1 or 2, X is a halogen atom, Y is NH₂ or phthalimido and R₁₁ is a (C₁-C₄)alkyl group.

7. A compound of claim 6 wherein Y is NH₂.

8. A compound of formula (I) or a pharmaceutically acceptable salt thereof as claimed in any one of claims 1 to 7 for use in therapy.

9. A compound or pharmaceutically acceptable salt of claim 8 for use in the treatment of digestive tract diseases.

10. A pharmaceutical composition comprising, as active ingredient, a compound of formula I or a pharmaceutically acceptable salt thereof as claimed in any one of claims 1 to 8 and a pharmaceutically acceptable carrier.

11. The use of a compound of formula I or a pharmaceutically acceptable salt as claimed in any one of claims 1 to 8 in the manufacture of a medicament for the treatment of digestive tract diseases.

12. Use according to claim 11 wherein the digestive tract disease is chronic gastritis, postgastrectomy syndrome, gastrointestinal symptoms associated with peptic ulcer, reflux esophagitis, irritiable bowel syndrome or spurious ileus.

13. A process of preparing a compound of claim 1, which comprises oxidizing 2,5-dihydrofuran to form 3-oxa-1, 5-pentanedial, reacting 3-oxa-1,5-pentanedial with substituted or unsubstituted benzylamine and acetonedicarboxylic acid to give 9-substituted or unsubstituted benzyl-3-oxa-9-azabicyclo[3.3.1]nonan-7-one, converting the carbonyl group on said oxazabicyclo ring to the oxime, reducing the oxime to give an endo-7-amino-9-substituted or unsubstituted benzyl-3-oxa-9-azabicyclo[3.3.1]nonane and reacting the resulting amino compound with a compound of formula ArCOOH wherein Ar is as defined in claim 1 or a reactive derivative thereof.
